# EUROPEAN PATENT APPLICATION

(11) **EP 3 998 108 A1**
(43) Date of publication of application: **18.05.2022**
(21) Application number: 20841146.2
(22) Date of filing: 10.07.2020
(51) Int. Cl.: A61P 21/04, A61P 43/00, C12N 5/10, C12N 15/113, A61K 31/712

(54) **ANTISENSE OLIGONUCLEOTIDE CAPABLE OF ALTERING SPLICING OF DUX4 PRE-MRNA**

(30) Priority: 12.07.2019 JP 2019129735
(71) Applicant: Daiichi Sankyo Company, Limited, Tokyo 103-8426 (JP); TOKAI UNIVERSITY EDUCATIONAL SYSTEM, Tokyo 151-0063 (JP)
(72) Inventor: KOIZUMI, Makoto, Tokyo 103-8426 (JP); NAKAMURA, Akifumi, Tokyo 103-8426 (JP); KATAGIRI, Takahiro, Tokyo 103-8426 (JP); MITSUHASHI, Hiroaki, Hiratsuka-shi, Kanagawa 259-1292 (JP)
(74) Representative: J A Kemp LLP
(86) International application number: PCT/JP2020/026950
(87) International publication number: WO 2021/010301

(57) **Abstract**

The present invention aims at establishing a novel therapy for facioscapulohumeral muscular dystrophy.

An oligonucleotide or a pharmaceutically acceptable salt thereof, wherein the oligonucleotide comprises an oligonucleotide of 15-30 bases consisting of a nucleotide sequence complementary to the region of nucleotide Nos. 502-556 or 578-612 of DUX4-fl mRNA consisting of the nucleotide sequence as shown in SEQ ID NO: 1; the 5' and/or 3' end of the oligonucleotide may be chemically modified; and the oligonucleotide is capable of switching the splice form of the DUX4 gene from DUX4-fl to DUX4-s. A pharmaceutical drug comprising the above oligonucleotide or a pharmaceutically acceptable salt thereof (e.g. therapeutic for facioscapulohumeral muscular dystrophy).

SEQUENCE LISTING

<110>  DAIICHI SANKYO COMPANY, LIMITED
       TOKAI UNIVERSITY EDUCATIONAL SYSTEM

<120>  Antisense oligonucleotides for changing splicing in DUX4 pre-mRNA

<130>  FP-283PCT

<150>  JP2019-129735
<151>  2019-07-12

<160>  102

<170>  PatentIn version 3.5

<210>  1
<211>  1655
<212>  DNA
<213>  Homo sapiens

<400>  1
atggccctcc cgacaccctc ggacagcacc ctccccgcgg aagcccgggg acgaggacgg      60

cgacggagac tcgtttggac cccgagccaa agcgaggccc tgcgagcctg ctttgagcgg     120

aacccgtacc cgggcatcgc caccagagaa cggctggccc aggccatcgg cattccggag     180

cccagggtcc agatttggtt tcagaatgag aggtcacgcc agctgaggca gcaccggcgg     240

gaatctcggc cctggcccgg gagacgcggc ccgccagaag gccggcgaaa gcggaccgcc     300

gtcaccggat cccagaccgc cctgctcctc cgagcctttg agaaggatcg ctttccaggc     360

atcgccgccc gggaggagct ggccagagag acgggcctcc cggagtccag gattcagatc     420

tggtttcaga atcgaagggc caggcacccg ggacagggtg gcagggcgcc cgcgcaggca     480

ggcggcctgt gcagcgcggc ccccggcggg ggtcaccctg ctccctcgtg ggtcgccttc     540

gcccacaccg gcgcgtgggg aacggggctt cccgcacccc acgtgccctg cgcgcctggg     600

gctctcccac agggggcttt cgtgagccag gcagcgaggg ccgcccccgc gctgcagccc     660

agccaggccg cgccggcaga ggggatctcc caacctgccc cggcgcgcgg ggatttcgcc     720

tacgccgccc cggctcctcc ggacggggcg ctctcccacc ctcaggctcc tcgctggcct     780

ccgcacccgg gcaaaagccg ggaggaccgg gacccgcagc gcgacggcct gccgggcccc     840

tgcgcggtgg cacagcctgg gcccgctcaa gcggggccgc agggccaagg ggtgcttgcg     900

ccacccacgt cccaggggag tccgtggtgg ggctggggcc ggggtcccca ggtcgccggg     960

gcggcgtggg aaccccaagc cggggcagct ccacctcccc agcccgcgcc cccggacgcc    1020

tccgcctccg cgcggcaggg gcagatgcaa ggcatcccgg cgccctccca ggcgctccag    1080

gagccggcgc cctggtctgc actcccctgc ggcctgctgc tggatgagct cctggcgagc    1140

ccggagtttc tgcagcaggc gcaacctctc ctagaaacgg aggccccggg ggagctggag    1200

gcctcggaag aggccgcctc gctggaagca cccctcagcg aggaagaata ccgggctctg    1260

ctggaggagc tttaggacgc ggggttggga cggggtcggg tggttcgggg cagggcggtg 1320

gcctctcttt cgcggggaac acctggctgg ctacggaggg gcgtgtctcc gccccgcccc 1380

ctccaccggg ctgaccggcc tgggattcct gccttctagg tctaggcccg gtgagagact 1440

ccacaccgcg gagaactgcc attctttcct gggcatcccg gggatcccag agccggccca 1500

ggtaccagca gacctgcgcg cagtgcgcac cccggctgac gtgcaaggga gctcgctggc 1560

ctctctgtgc ccttgttctt ccgtgaaatt ctggctgaat gtctcccccc accttccgac 1620

gctgtctagg caaacctgga ttagagttac atctc 1655

<210> 2
<211> 18
<212> DNA
<213> Artificial Sequence

<220>
<223> antisense oligonucleotide

<400> 2
ctgtgggaga gccccagg 18

<210> 3
<211> 18
<212> DNA
<213> Artificial Sequence

<220>
<223> antisense oligonucleotide

<400> 3
agggtgaccc ccgccggg 18

<210> 4
<211> 18
<212> DNA
<213> Artificial Sequence

<220>
<223> antisense oligonucleotide

<400> 4
agcagggtga cccccgcc 18

<210> 5
<211> 18
<212> DNA
<213> Artificial Sequence

<220>
<223> antisense oligonucleotide

<400> 5
gagcagggtg acccccgc 18

<210>  6
<211>  18
<212>  DNA
<213>  Artificial Sequence

<220>
<223>  antisense oligonucleotide

<400>  6
ggagcagggt gaccccg                                                    18


<210>  7
<211>  18
<212>  DNA
<213>  Artificial Sequence

<220>
<223>  antisense oligonucleotide

<400>  7
gggagcaggg tgaccccc                                                   18


<210>  8
<211>  18
<212>  DNA
<213>  Artificial Sequence

<220>
<223>  antisense oligonucleotide

<400>  8
agggagcagg gtgacccc                                                   18


<210>  9
<211>  18
<212>  DNA
<213>  Artificial Sequence

<220>
<223>  antisense oligonucleotide

<400>  9
gagggagcag ggtgaccc                                                   18


<210>  10
<211>  18
<212>  DNA
<213>  Artificial Sequence

<220>
<223>  antisense oligonucleotide

<400>  10
cgagggagca gggtgacc                                                   18


<210>  11
<211>  18

<212>  DNA
<213>  Artificial Sequence

<220>
<223>  antisense oligonucleotide

<400>  11
acgagggagc agggtgac                                                    18


<210>  12
<211>  18
<212>  DNA
<213>  Artificial Sequence

<220>
<223>  antisense oligonucleotide

<400>  12
cacgagggag cagggtga                                                    18


<210>  13
<211>  18
<212>  DNA
<213>  Artificial Sequence

<220>
<223>  antisense oligonucleotide

<400>  13
ccacgaggga gcagggtg                                                    18


<210>  14
<211>  18
<212>  DNA
<213>  Artificial Sequence

<220>
<223>  antisense oligonucleotide

<400>  14
cccacgaggg agcagggt                                                    18


<210>  15
<211>  18
<212>  DNA
<213>  Artificial Sequence

<220>
<223>  antisense oligonucleotide

<400>  15
acccacgagg gagcaggg                                                    18


<210>  16
<211>  18
<212>  DNA
<213>  Artificial Sequence

<220>
<223>  antisense oligonucleotide

<400>  16
gacccacgag ggagcagg                                                    18


<210>  17
<211>  18
<212>  DNA
<213>  Artificial Sequence

<220>
<223>  antisense oligonucleotide

<400>  17
cgacccacga gggagcag                                                    18


<210>  18
<211>  18
<212>  DNA
<213>  Artificial Sequence

<220>
<223>  antisense oligonucleotide

<400>  18
gcgacccacg agggagca                                                    18


<210>  19
<211>  18
<212>  DNA
<213>  Artificial Sequence

<220>
<223>  antisense oligonucleotide

<400>  19
ggcgacccac gagggagc                                                    18


<210>  20
<211>  18
<212>  DNA
<213>  Artificial Sequence

<220>
<223>  antisense oligonucleotide

<400>  20
aggcgaccca cgagggag                                                    18


<210>  21
<211>  18
<212>  DNA
<213>  Artificial Sequence

<220>
<223>  antisense oligonucleotide

<400>  21
aaggcgaccc acgaggga                                                     18


<210>  22
<211>  18
<212>  DNA
<213>  Artificial Sequence

<220>
<223>  antisense oligonucleotide

<400>  22
gaaggcgacc cacgaggg                                                     18


<210>  23
<211>  18
<212>  DNA
<213>  Artificial Sequence

<220>
<223>  antisense oligonucleotide

<400>  23
cgaaggcgac ccacgagg                                                     18


<210>  24
<211>  18
<212>  DNA
<213>  Artificial Sequence

<220>
<223>  antisense oligonucleotide

<400>  24
gcgaaggcga cccacgag                                                     18


<210>  25
<211>  18
<212>  DNA
<213>  Artificial Sequence

<220>
<223>  antisense oligonucleotide

<400>  25
ggcgaaggcg acccacga                                                     18


<210>  26
<211>  18
<212>  DNA
<213>  Artificial Sequence

<220>
<223>  antisense oligonucleotide

<400>  26
gggcgaaggc gacccacg                                                     18

<210>  27
<211>  18
<212>  DNA
<213>  Artificial Sequence

<220>
<223>  antisense oligonucleotide

<400>  27
tgggcgaagg cgacccac                                                    18


<210>  28
<211>  18
<212>  DNA
<213>  Artificial Sequence

<220>
<223>  antisense oligonucleotide

<400>  28
gtgggcgaag gcgaccca                                                    18


<210>  29
<211>  18
<212>  DNA
<213>  Artificial Sequence

<220>
<223>  antisense oligonucleotide

<400>  29
tgtgggcgaa ggcgaccc                                                    18


<210>  30
<211>  18
<212>  DNA
<213>  Artificial Sequence

<220>
<223>  antisense oligonucleotide

<400>  30
gtgtgggcga aggcgacc                                                    18


<210>  31
<211>  18
<212>  DNA
<213>  Artificial Sequence

<220>
<223>  antisense oligonucleotide

<400>  31
ggtgtgggcg aaggcgac                                                    18


<210>  32
<211>  18

<212>  DNA
<213>  Artificial Sequence

<220>
<223>  antisense oligonucleotide

<400>  32
cggtgtgggc gaaggcga                                                    18


<210>  33
<211>  18
<212>  DNA
<213>  Artificial Sequence

<220>
<223>  antisense oligonucleotide

<400>  33
ccggtgtggg cgaaggcg                                                    18


<210>  34
<211>  18
<212>  DNA
<213>  Artificial Sequence

<220>
<223>  antisense oligonucleotide

<400>  34
gccggtgtgg gcgaaggc                                                    18


<210>  35
<211>  18
<212>  DNA
<213>  Artificial Sequence

<220>
<223>  antisense oligonucleotide

<400>  35
cgccggtgtg ggcgaagg                                                    18


<210>  36
<211>  18
<212>  DNA
<213>  Artificial Sequence

<220>
<223>  antisense oligonucleotide

<400>  36
gcgccggtgt gggcgaag                                                    18


<210>  37
<211>  18
<212>  DNA
<213>  Artificial Sequence

<220>
<223>  antisense oligonucleotide

<400>  37
cgcgccggtg tgggcgaa                                                    18


<210>  38
<211>  18
<212>  DNA
<213>  Artificial Sequence

<220>
<223>  antisense oligonucleotide

<400>  38
acgcgccggt gtgggcga                                                    18


<210>  39
<211>  18
<212>  DNA
<213>  Artificial Sequence

<220>
<223>  antisense oligonucleotide

<400>  39
gagccccagg cgcgcagg                                                    18


<210>  40
<211>  18
<212>  DNA
<213>  Artificial Sequence

<220>
<223>  antisense oligonucleotide

<400>  40
caggcgcgca gggcacgt                                                    18


<210>  41
<211>  18
<212>  DNA
<213>  Artificial Sequence

<220>
<223>  antisense oligonucleotide

<400>  41
cccaggcgcg cagggcac                                                    18


<210>  42
<211>  18
<212>  DNA
<213>  Artificial Sequence

<220>
<223>  antisense oligonucleotide

<400>  42
ccccaggcgc gcagggca                                                    18


<210>  43
<211>  18
<212>  DNA
<213>  Artificial Sequence

<220>
<223>  antisense oligonucleotide

<400>  43
agccccaggc gcgcaggg                                                    18


<210>  44
<211>  18
<212>  DNA
<213>  Artificial Sequence

<220>
<223>  antisense oligonucleotide

<400>  44
gagccccagg cgcgcagg                                                    18


<210>  45
<211>  18
<212>  DNA
<213>  Artificial Sequence

<220>
<223>  antisense oligonucleotide

<400>  45
gagagcccca ggcgcgca                                                    18


<210>  46
<211>  18
<212>  DNA
<213>  Artificial Sequence

<220>
<223>  antisense oligonucleotide

<400>  46
ggagagcccc aggcgcgc                                                    18


<210>  47
<211>  18
<212>  DNA
<213>  Artificial Sequence

<220>
<223>  antisense oligonucleotide

<400>  47
tgggagagcc ccaggcgc                                                    18

<210>  48
<211>  18
<212>  DNA
<213>  Artificial Sequence

<220>
<223>  antisense oligonucleotide

<400>  48
gtgggagagc cccaggcg                                                    18


<210>  49
<211>  17
<212>  DNA
<213>  Artificial Sequence

<220>
<223>  antisense oligonucleotide

<400>  49
agcagggtga cccccgc                                                     17


<210>  50
<211>  17
<212>  DNA
<213>  Artificial Sequence

<220>
<223>  antisense oligonucleotide

<400>  50
gagcagggtg acccccg                                                     17


<210>  51
<211>  17
<212>  DNA
<213>  Artificial Sequence

<220>
<223>  antisense oligonucleotide

<400>  51
ggagcagggt gaccccc                                                     17


<210>  52
<211>  17
<212>  DNA
<213>  Artificial Sequence

<220>
<223>  antisense oligonucleotide

<400>  52
gggagcaggg tgacccc                                                     17


<210>  53
<211>  17

<212>  DNA
<213>  Artificial Sequence

<220>
<223>  antisense oligonucleotide

<400>  53
cgagggagca gggtgac                                                      17


<210>  54
<211>  17
<212>  DNA
<213>  Artificial Sequence

<220>
<223>  antisense oligonucleotide

<400>  54
acgagggagc agggtga                                                      17


<210>  55
<211>  17
<212>  DNA
<213>  Artificial Sequence

<220>
<223>  antisense oligonucleotide

<400>  55
acccacgagg gagcagg                                                      17


<210>  56
<211>  17
<212>  DNA
<213>  Artificial Sequence

<220>
<223>  antisense oligonucleotide

<400>  56
gacccacgag ggagcag                                                      17


<210>  57
<211>  17
<212>  DNA
<213>  Artificial Sequence

<220>
<223>  antisense oligonucleotide

<400>  57
cgacccacga gggagca                                                      17


<210>  58
<211>  17
<212>  DNA
<213>  Artificial Sequence

<220>
<223>  antisense oligonucleotide

<400>  58
aaggcgaccc acgaggg                                                      17


<210>  59
<211>  17
<212>  DNA
<213>  Artificial Sequence

<220>
<223>  antisense oligonucleotide

<400>  59
gaaggcgacc cacgagg                                                      17


<210>  60
<211>  17
<212>  DNA
<213>  Artificial Sequence

<220>
<223>  antisense oligonucleotide

<400>  60
cgaaggcgac ccacgag                                                      17


<210>  61
<211>  17
<212>  DNA
<213>  Artificial Sequence

<220>
<223>  antisense oligonucleotide

<400>  61
gtgggcgaag gcgaccc                                                      17


<210>  62
<211>  17
<212>  DNA
<213>  Artificial Sequence

<220>
<223>  antisense oligonucleotide

<400>  62
tgtgggcgaa ggcgacc                                                      17


<210>  63
<211>  17
<212>  DNA
<213>  Artificial Sequence

<220>
<223>  antisense oligonucleotide

<400>  63
gtgtgggcga aggcgac                                                      17


<210>  64
<211>  17
<212>  DNA
<213>  Artificial Sequence

<220>
<223>  antisense oligonucleotide

<400>  64
ggtgtgggcg aaggcga                                                      17


<210>  65
<211>  16
<212>  DNA
<213>  Artificial Sequence

<220>
<223>  antisense oligonucleotide

<400>  65
   gcagggtgac ccccgc                                                        16


<210>  66
<211>  16
<212>  DNA
<213>  Artificial Sequence

<220>
<223>  antisense oligonucleotide

<400>  66
   agcagggtga cccccg                                                        16


<210>  67
<211>  16
<212>  DNA
<213>  Artificial Sequence

<220>
<223>  antisense oligonucleotide

<400>  67
gagcagggtg accccc                                                       16


<210>  68
<211>  16
<212>  DNA
<213>  Artificial Sequence

<220>
<223>  antisense oligonucleotide

<400>  68
ggagcagggt gacccc                                                       16

<210>  69
<211>  16
<212>  DNA
<213>  Artificial Sequence

<220>
<223>  antisense oligonucleotide

<400>  69
gggagcaggg tgaccc                                                        16


<210>  70
<211>  16
<212>  DNA
<213>  Artificial Sequence

<220>
<223>  antisense oligonucleotide

<400>  70
gagggagcag ggtgac                                                        16


<210>  71
<211>  16
<212>  DNA
<213>  Artificial Sequence

<220>
<223>  antisense oligonucleotide

<400>  71
cgagggagca gggtga                                                        16


<210>  72
<211>  16
<212>  DNA
<213>  Artificial Sequence

<220>
<223>  antisense oligonucleotide

<400>  72
acgagggagc agggtg                                                        16


<210>  73
<211>  16
<212>  DNA
<213>  Artificial Sequence

<220>
<223>  antisense oligonucleotide

<400>  73
cccacgaggg agcagg                                                        16


<210>  74
<211>  16

<212>  DNA
<213>  Artificial Sequence

<220>
<223>  antisense oligonucleotide

<400>  74
acccacgagg gagcag                                                       16


<210>  75
<211>  16
<212>  DNA
<213>  Artificial Sequence

<220>
<223>  antisense oligonucleotide

<400>  75
gacccacgag ggagca                                                       16


<210>  76
<211>  16
<212>  DNA
<213>  Artificial Sequence

<220>
<223>  antisense oligonucleotide

<400>  76
cgacccacga gggagc                                                       16


<210>  77
<211>  16
<212>  DNA
<213>  Artificial Sequence

<220>
<223>  antisense oligonucleotide

<400>  77
aggcgaccca cgaggg                                                       16


<210>  78
<211>  16
<212>  DNA
<213>  Artificial Sequence

<220>
<223>  antisense oligonucleotide

<400>  78
aaggcgaccc acgagg                                                       16


<210>  79
<211>  16
<212>  DNA
<213>  Artificial Sequence

<220>
<223>  antisense oligonucleotide

<400>  79
gaaggcgacc cacgag                                                       16


<210>  80
<211>  16
<212>  DNA
<213>  Artificial Sequence

<220>
<223>  antisense oligonucleotide

<400>  80
cgaaggcgac ccacga                                                       16


<210>  81
<211>  16
<212>  DNA
<213>  Artificial Sequence

<220>
<223>  antisense oligonucleotide

<400>  81
tgggcgaagg cgaccc                                                       16


<210>  82
<211>  16
<212>  DNA
<213>  Artificial Sequence

<220>
<223>  antisense oligonucleotide

<400>  82
gtgggcgaag gcgacc                                                       16


<210>  83
<211>  16
<212>  DNA
<213>  Artificial Sequence

<220>
<223>  antisense oligonucleotide

<400>  83
tgtgggcgaa ggcgac                                                       16


<210>  84
<211>  16
<212>  DNA
<213>  Artificial Sequence

<220>
<223>  antisense oligonucleotide

<400>  84
gtgtgggcga aggcga                                                        16


<210>  85
<211>  16
<212>  DNA
<213>  Artificial Sequence

<220>
<223>  antisense oligonucleotide

<400>  85
ggtgtgggcg aaggcg                                                        16


<210>  86
<211>  21
<212>  DNA
<213>  Artificial Sequence

<220>
<223>  primer

<400>  86
cgcgtccgtc cgtgaaattc c                                                  21


<210>  87
<211>  28
<212>  DNA
<213>  Artificial Sequence

<220>
<223>  primer

<400>  87
caggggatat tgtgacatat ctctgcac                                           28


<210>  88
<211>  25
<212>  DNA
<213>  Artificial Sequence

<220>
<223>  primer

<400>  88
gaattctgcc accatggccc tcccg                                              25


<210>  89
<211>  26
<212>  DNA
<213>  Artificial Sequence

<220>
<223>  primer

<400>  89
ctcgagctat aggatccaca gggagg                                             26

<210>  90
<211>  29
<212>  DNA
<213>  Artificial Sequence

<220>
<223>  primer

<400>  90
ggattcagat ctggtttcag aatcgaagg                                          29


<210>  91
<211>  30
<212>  DNA
<213>  Artificial Sequence

<220>
<223>  primer

<400>  91
ccaggagatg taactctaat ccaggtttgc                                         30


<210>  92
<211>  24
<212>  DNA
<213>  Artificial Sequence

<220>
<223>  primer

<400>  92
cgacgttgta aaacgacggc cagt                                               24


<210>  93
<211>  24
<212>  DNA
<213>  Artificial Sequence

<220>
<223>  primer

<400>  93
ggaaacagct atgaccatga ttac                                               24


<210>  94
<211>  20
<212>  DNA
<213>  Artificial Sequence

<220>
<223>  primer

<400>  94
tggaaatcaa gtggcaaaaa                                                    20


<210>  95
<211>  18

<212>  DNA
<213>  Artificial Sequence

<220>
<223>  primer

<400>  95
ctgcatgtgg acgtggac                                                    18


<210>  96
<211>  20
<212>  DNA
<213>  Artificial Sequence

<220>
<223>  primer

<400>  96
gcgttcacct cttttccaag                                                  20


<210>  97
<211>  20
<212>  DNA
<213>  Artificial Sequence

<220>
<223>  primer

<400>  97
gccatgtgga tttctcgttt                                                  20


<210>  98
<211>  20
<212>  DNA
<213>  Artificial Sequence

<220>
<223>  primer

<400>  98
acccatcact ggactggtgt                                                  20


<210>  99
<211>  20
<212>  DNA
<213>  Artificial Sequence

<220>
<223>  primer

<400>  99
cacatcctca aagagcctga                                                  20


<210>  100
<211>  18
<212>  DNA
<213>  Artificial Sequence

<220>
<223>  antisense oligonucleotide

<400>  100
tgtgggcgaa ggcgaccc                                                    18


<210>  101
<211>  18
<212>  DNA
<213>  Artificial Sequence

<220>
<223>  antisense oligonucleotide

<400>  101
tgugggcgaa ggcgaccc                                                    18


<210>  102
<211>  18
<212>  DNA
<213>  Artificial Sequence

<220>
<223>  antisense oligonucleotide

<400>  102
ugtgggcgaa ggcgaccc                                                    18

## Description

### TECHNICAL FIELD

The present invention relates to an antisense oligonucleotide capable of altering the splicing of DUX4 pre-mRNA.

### BACKGROUND ART

Facioscapulohumeral muscular dystrophy (FSHD) is a muscular dystrophy characterized by strong atrophy and weakness of the muscles in the face/cheek, around the shoulder blades and in the upper arms (Non-Patent Document No. 1: Tawil R. and Van Der Maarel S.M. Muscle Nerve 2006, 34:1-15). The age of onset is very wide from 0 to 60's. Generally, FSHD begins to manifest at around ages 10-19 with symptoms such as difficulty in raising arms, inability to blow a whistle, poor facial expression, etc. Although symptoms progress slowly, the disorder descends and extend gradually to legs, often leading to difficulty in walking. Further, the degree of muscle disorder is often asymmetric. Muscle pathological findings include necrosis and regeneration of muscle fibers as observed in muscular dystrophy in general, and sometimes infiltration of inflammatory cells (Non-Patent Document No. 2: Arahata et al., Muscle Nerve 1995, 2:S56-S66). In addition to the symptoms in the skeletal muscle, combination of neural hearing loss or retinopathy is frequently observed (Non-Patent Document No. 3: Padberg et al., Muscle Nerve 1995, 2:S73-S80). It is considered that the frequency of FSHD is the third in hereditary muscle diseases after Duchenne muscular dystrophy and myotonic dystrophy; and the prevalence of FSHD is reported to be 1/14,000 to 1/20,000 in the United States and Europe (Non-Patent Document No. 4: Padberg et al., Muscle Nerve 1995, 2:S81-S84; Flanigan et al., Neuromuscul Disord 2001, 11:525-529; Non-Patent Document No. 5: Mostacciuolo et al., Clin Genet 2009, 75:550-555; Norwood et al, Brain 2009, 132:3175-3186; Non-Patent Document No. 6: Norwood et al, Brain 2009, 132:3175-3186; and Non-Patent Document No. 7: Deenen et al., Neurology 2014, 83:1056-1059).

Although the genetic predisposition causing FSHD is very complicated, the theory of "derepression of DUX4 gene" published by Lemmers et al., in 2010 is now widely accepted (Non-Patent Document No. 8: Lemmers et al., Science 2010, 329:1650-1653). FSHD is inherited in an autosomal dominant manner and is linked to the end of the long arm of chromosome 4 (4q35 region) (Non-Patent Document No. 9: Wijmenga et al., Nat Genet 1992, 2:26-30). In this 4q35 region, there is a region in which 3.3 kb sequence (called D4Z4) is repeated (D4Z4 repeat). Normally, D4Z4 is repeated 11 to 100 times and this 4q35 region takes a state of heterochromatin which is highly DNA-methylated In about 95% of FSHD patients, the D4Z4 repeat in one copy of chromosome 4 is shortened to 10 reprats or less, resulting in reduction of DNA methylation in the 4q35 region (Non-Patent Document No. 10: van Overveld et al., Nat Genet 2003, 35:315-317). The type of FSHD in which this shortened D4Z4 repeat is observed is called FSHD1. Although the number of D4Z4 is normal in about 5% of FSHD patients, reduction of DNA methylation is occurring; this type of FSHD is called FSHD2. At present, it is known that the reduction of methylation in FSHD2 is caused by a loss-of-function mutation in the SMCHD gene involved in the methylation of the D4Z4 repeat (Non-Patent Document No. 11: Lemmers et al., Nat Genet 2012, 44:1370-1374).

An open reading frame (ORF) encoding a transcription factor protein exists in the D4Z4 sequence and is called DUX4 (Non-Patent Document No. 12: Gabriels et al., Gene 1999, 236:25-32). Expression of DUX4 in the skeletal muscle occurs at high frequency only when the following two conditions are satisfied. The first condition is that DNA methylation is reduced in the D4Z4 region and the heterochromatin structure is loosened. The second condition is that the haplotype (called 4qA or 4qB) present in the 3' end of the D4Z4 repeat is 4qA (Non-Patent Document No. 13: Lemmers et al., Am J Hum Genet 2007, 81:884-894). The 4qA sequence contains a polyadenylation signal sequence (ATTAAA), which stabilizes mRNA of DUX4 (Non-Patent Document No. 14: Lemmers et al., Science 2010, 329:1650-1653). Since the 4qB sequence does not contain a polyadenylation signal sequence, individuals with the 4qB allele and the D4Z4 repeat shortened to 10 or less do not present with symptoms of FSHD

The DUX4 gene encodes a transcription factor and is capable of generating transcription products DUX4-fl and DUX4-s through alternative splicing (Non-Patent Document No. 15: Snider et al., PLoS Genet 2010, 6:e1001181). Full-length DUX4-fl is expressed at high frequency in the FSHD patients (Non-Patent Document No. 16: Jones et al., Hum Mol Genet 2012, 21:4419-4430), and this protein exerts a strong transcriptional activity via its C-terminal transcriptional activation domain (Non-Patent Document No. 17: Choi et al., Nucleic Acids Res 2016, 44:5161-5173; Non-Patent Document No. 18: Mitsuhashi et al., Biol Open 2018, 7:bio033977). On the other hand, short type DUX4-s slightly expressed even in healthy individuals, however, does not have a transcriptional activation domain and thus does not function as a transcription factor. The DUX4-fl protein activates the expression of genes such as PRAMEF2, TRIM43, and ZSCAN4 expressed in early embryos, or testis, or the like (Non-Patent Document No. 19: Geng et al., Dev Cell 2012, 22:38-51). It has been reported that the expression of DUX4-fl causes apoptosis (Non-Patent Document No. 20: Kowaljow et al., Neuromuscul Disord 2007, 17:611-623; Non-Patent Document No. 21: Wallace et al., Ann Neurol 2011, 69:540-552), abnormal muscle development (Non-Patent Document No. 22: Bosnakovski et al., EMBO J 2008, 27:2766-2779; Non-Patent Document No. 23: Banerji et al., Nat Commun 2017, 8:2152), inhibition of nonsense-mediated mRNA decay (Non-Patent Document No. 24: Feng et al., eLife 2015, 4:e04996), formation of intracellular double-stranded RNA (Non-Patent Document No. 25: Shadle et al., PLoS Genet 2017, 13:e1006658), protein aggregation (Non-Patent Document No. 26: Homma et al., Ann Clin Transl Neurol 2015, 2:151-166) and so forth. It has been shown that DUX4-fl induces cytotoxicity and symptoms similar to those obserbed in FSHD even in the following animal models engineered to express DUX4-fl: Drosophila (Non-Patent Document No. 27: Jones et al., PLoS One 2016, 11:e0150938), Xenopus (Non-Patent Document No. 28: Wuebbles et al. Int J Clin Exp Pathol 2010, 3:386-400), zebrafish (Non-Patent Document No. 29: Mitsuhashi et al., Hum Mol Genet 2013, 22:568-577) and mouse (Non-Patent Document No. 30: Jones and Jones, PLoS One 2018, 13:e0192657; Non-Patent Document No. 31: Bosnakovski et al., Nat Commun 2017, 8:550). Therefore, it is considered that DUX4-fl is involved in the onset of FSHD. Further, as a physiological function of DUX4, it has been shown that DUX4 is a master transcription factor which is expressed specifically at the four-cell stage during early embryogenesis and activates a group of genes specifically expressed at cleavage stage, including the above-mentioned ZSCAN4, etc. However, details of the biological significance have not been elucidated yet (Non-Patent Document No. 32: De laco et al., Nat Genet 2017, 49:941-945).

DUX4-fl and DUX4-s have a common DNA binding domain in the N-terminal region. Therefore, when DUX4-fl and DUX4-s are present at the same time, it is considered that they bind to the same DNA elements competitively. In experiments with cultured cells, activation of luciferase by DUX4-fl was suppressed to about 20% when cells were co-transfected with DUX4-fl and DUX4-s at the 1:1 ratio (Non-Patent Document No. 34: Geng et al., Dev Cell 2012, 22:38-51). Further, in the experiments using zebrafish co-transfected with DUX4-fl and DUX4-s at the1:20 ratio, muscular dystrophy-like symptoms caused by DUX4-fl were alleviated (Non-Patent Document No. 35: Mitsuhashi et al., Hum Mol Genet 2013, 22:568-577). Since DUX4-s does not have transcription promoting activity, it is considered that competitive inhibition against the target gene must have occurred.

### PRIOR ART LITERATURE

### Non-Patent Documents

Non-Patent Document No. 1: Tawil R. and Van Der Maarel S.M. Muscle Nerve 2006, 34:1-15
Non-Patent Document No. 2: Arahata et al., Muscle Nerve 1995, 2:S56-S66
Non-Patent Document No. 3: Padberg et al., Muscle Nerve 1995, 2:S73-S80
Non-Patent Document No. 4: Padberg et al., Muscle Nerve 1995, 2:S81-S84; Flanigan et al., Neuromuscul Disord 2001, 11:525-529
Non-Patent Document No. 5: Mostacciuolo et al., Clin Genet 2009, 75:550-555; Norwood et al, Brain 2009, 132:3175-3186
Non-Patent Document No. 6: Norwood et al, Brain 2009, 132:3175-3186
Non-Patent Document No. 7: Deenen et al., Neurology 2014, 83:1056-1059
Non-Patent Document No. 8: Lemmers et al., Science 2010, 329:1650-1653
Non-Patent Document No. 9: Wijmenga et al., Nat Genet 1992, 2:26-30
Non-Patent Document No. 10: van Overveld et al., Nat Genet 2003, 35:315-317
Non-Patent Document No. 11: Lemmers et al., Nat Genet 2012, 44:1370-1374
Non-Patent Document No. 12: Gabriels et al., Gene 1999, 236:25-32.
Non-Patent Document No. 13: Lemmers et al., Am J Hum Genet 2007, 81:884-894
Non-Patent Document No. 14: Lemmers et al., Science 2010, 329:1650-1653
Non-Patent Document No. 15: Snider et al., PLoS Genet 2010, 6:e1001181
Non-Patent Document No. 16: Jones et al., Hum Mol Genet 2012, 21:4419-4430
Non-Patent Document No. 17: Choi et al., Nucleic Acids Res 2016, 44:5161-5173
Non-Patent Document No. 18: Mitsuhashi et al., Biol Open 2018, 7:bio033977
Non-Patent Document No. 19: Geng et al., Dev Cell 2012, 22:38-51
Non-Patent Document No. 20: Kowaljow et al., Neuromuscul Disord 2007, 17:611-623
Non-Patent Document No. 21: Wallace et al., Ann Neurol 2011, 69:540-552
Non-Patent Document No. 22: Bosnakovski et al., EMBO J 2008, 27:2766-2779
Non-Patent Document No. 23: Banerji et al., Nat Commun 2017, 8:2152
Non-Patent Document No. 24: Feng et al., eLife 2015, 4:e04996
Non-Patent Document No. 25: Shadle et al., PLoS Genet 2017, 13:e1006658
Non-Patent Document No. 26: Homma et al., Ann Clin Transl Neurol 2015, 2:151-166
Non-Patent Document No. 27: Jones et al., PLoS One 2016, 11:e0150938
Non-Patent Document No. 28: Wuebbles et al. Int J Clin Exp Pathol 2010, 3:386-400
Non-Patent Document No. 29: Mitsuhashi et al., Hum Mol Genet 2013, 22:568-577
Non-Patent Document No. 30: Jones and Jones, PLoS One 2018, 13:e0192657
Non-Patent Document No. 31: Bosnakovski et al., Nat Commun 2017, 8:550
Non-Patent Document No. 32: De laco et al., Nat Genet 2017, 49:941-945
Non-Patent Document No. 33: Hendrickson et al., Nat Genet 2017, 49:925-934
Non-Patent Document No. 34: Geng et al., Dev Cell 2012, 22:38-51
Non-Patent Document No. 35: Mitsuhashi et al., Hum Mol Genet 2013, 22:568-577

### DISCLOSURE OF THE INVENTION

### PROBLEM FOR SOLUTION BY THE INVENTION

It is an object of the present invention to establish a novel therapy for facioscapulohumeral muscular dystrophy.

### MEANS TO SOLVE THE PROBLEM

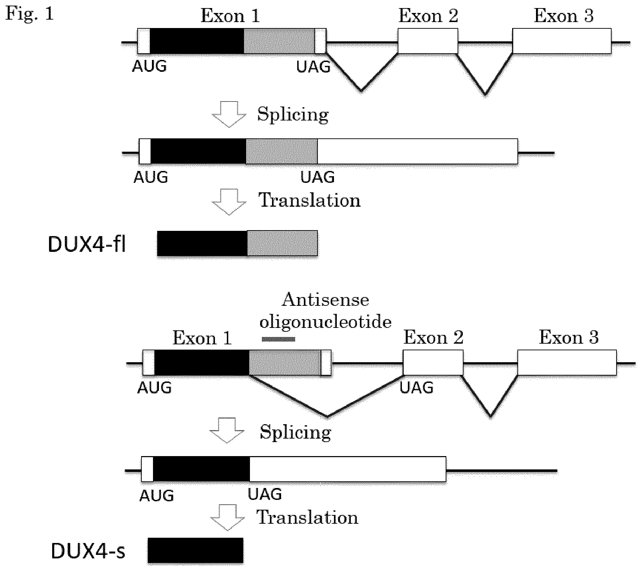

Since DUX4-fl and DUX4-s are different splicing isoforms transcribed from the DUX4 gene, it may potentially be possible to suppress cell death by decreasing the relative transcription level of DUX4-fl through switching the splice form of the DUX4 gene from DUX4-fl to DUX4-s with an antisense oligonucleotide (Fig. 1). The present invention has been achieved based on this idea.

A summary of the present invention is described as below.
(1) An oligonucleotide or a pharmaceutically acceptable salt thereof, wherein the oligonucleotide comprises an oligonucleotide of 15-30 bases consisting of a nucleotide sequence complementary to the region of nucleotide Nos. 502-556 or 578-612 of DUX4-fl mRNA consisting of the nucleotide sequence as shown in SEQ ID NO: 1; the 5' and/or 3' end of the oligonucleotide may be chemically modified; and the oligonucleotide is capable of switching the splice form of the DUX4 gene from DUX4-fl to DUX4-s.
(2) The oligonucleotide or a pharmaceutically acceptable salt thereof of (1) above, wherein the oligonucleotide comprises a sequence of at least 15 consecutive nucleotides in any one of the sequences as shown in SEQ ID NOS: 2-85 (wherein "t" may be "u", and "u" may be "t").
(3) The oligonucleotide or a pharmaceutically acceptable salt thereof of (1) or (2) above, wherein the oligonucleotide has 16-18 bases.
(4) The oligonucleotide or a pharmaceutically acceptable salt thereof of (3) above, wherein the oligonucleotide has 18 bases.
(5) The oligonucleotide or a pharmaceutically acceptable salt thereof of any one of (1) to (4) above, wherein at least one of the sugar and/or the phosphodiester bond constituting the oligonucleotide is modified.
(6) The oligonucleotide or a pharmaceutically acceptable salt thereof of (5) above, wherein the sugar constituting the oligonucleotide is D-ribofuranose and modification of the sugar is modification of the hydroxy group at 2'-position of D-ribofuranose.
(7) The oligonucleotide or a pharmaceutically acceptable salt thereof of (6) above, wherein modification of the sugar is 2'-O-alkylation and/or 2'-O,4'-C-alkylenation of D-ribofuranose.
(8) The oligonucleotide or a pharmaceutically acceptable salt thereof of (6) above, wherein modification of the sugar is 2'-O-methylation and/or 2'-O,4'-C-ethylenation of D-ribofuranose.
(9) The oligonucleotide or a pharmaceutically acceptable salt thereof of any one of (5) to (8) above, wherein modification of the phosphodiester bond is a phosphorothioate bond.
(10) The oligonucleotide or a pharmaceutically acceptable salt thereof of any one of (1) to (9) above, wherein the oligonucleotide is one having 15-30 bases consisting of a nucleotide sequence complementary to the region of nucleotide Nos. 506-549 of the nucleotide sequence as shown in SEQ ID NO: 1.
(11) The oligonucleotide or a pharmaceutically acceptable salt thereof of (10) above, wherein the oligonucleotide comprises a sequence of at least 15 consecutive nucleotides in any one of the sequences as shown in SEQ ID NOS: 5-31 (wherein "t" may be "u", and "u" may be "t").
(12) An oligonucleotide consisting of any one of the following sequences or a pharmaceutically acceptable salt thereof:
   HD-G^{mls}-G^{e2s}-G^{mls}-A^{mls}-G^{e2s}-C^{mls}-A^{mls}-G^{e2s}-G^{mls}-G^{mls}-T^{e2s}-G^{mls}-A^{mls}-C^{e2s}-C^{mls}-C^{mls}-C^{e2s}-C^{mlt}-H (DUX4-006);
   HO-G^{mls}-A^{e2s}-C^{mls}-C^{mls}-C^{e2s}-A^{mls}-C^{mls}-G^{e2s}-A^{mls}-G^{mls}-G^{e2s}-G^{mls}-A^{mls}-G^{e2s}-C^{mls}-A^{mls}-G^{e2s}-G^{mlt}-H (DUX4-009);
   HO-G^{mls}-A^{e2s}-A^{mls}-G^{mls}-G^{e2s}-C^{mls}-G^{mls}-A^{e2s}-C^{mls}-C^{mls}-C^{e2s}-A^{mls}-C^{mls}-G^{e2s}-A^{mls}-G^{mls}-G^{e2s}-G^{mlt}-H (DUX4-011);
   HO-G^{mls}-G^{e2s}-U^{mls}-G^{mls}-T^{e2s}-G^{mls}-G^{mls}-G^{e2s}-C^{mls}-G^{mls}-A^{e2s}-A^{mls}-G^{mls}-G^{e2s}-C^{mls}-G^{mls}-A^{e2s}-C^{mlt}-H (DUX4-014);
   HO-G^{mls}-A^{e2s-}G^{mls}-C^{mls}-A^{e2s}-G^{mls}-G^{mls}-G^{e2s}-U^{mls}-G^{mls}-A^{e2s}-C^{mls}-C^{mls}-C^{e2s}-C^{mls}-C^{mls}-G^{e2s}-C^{mlt}-H (DUX4-036);
   HO-G^{mls}-G^{e2s}-A^{mls}-G^{mls}-C^{e2s}-A^{mls}-G^{mls}-G^{e2s}-G^{mls}-U^{mls}-G^{e2s}-A^{mls}-C^{mls}-C^{e2s}-C^{mls}-C^{mls}-C^{e2s}-G^{mlt}-H (DUX4-037);
   HO-A^{mls}-C^{e2s}-G^{mls}-A^{mls}-G^{e2s}-G^{mls}-G^{mls}-A^{e2s}-G^{mls}-C^{mls}-A^{e2s}-G^{mls}-G^{mls}-G^{e2s}-U^{mls}-G^{mls}-A^{e2s}-C^{mlt}-H (DUX4-040);
   HO-C^{mls}-G^{e2s}-A^{mls}-C^{mls}-C^{e2s}-C^{mls}-A^{mls}-C^{e2s}-G^{mls}-A^{mls}-G^{e2s}-G^{mls}-G^{mls}-A^{e2s}-G^{mls}-C^{mls}-A^{e2s}-G^{mlt}-H (DUX4-044);
   HO-A^{mls}-A^{e2s}-G^{mls-}G^{mls}-C^{e2s}-G^{mls}-A^{mls-}C^{e2s-}C^{mls}-C^{mls}-A^{e2s-}C^{mls}-G^{mls}-A^{e2s}-G^{mls}-G^{mls}-G^{e2s}-A^{mlt}-H (DUX4-047);
   HO-C^{mls}-G^{e2s}-A^{mls}-A^{mls-}G^{e2s}-G^{mls}-C^{mls}-G^{e2s}-A^{mls}-C^{mls}-C^{e2s}-C^{mls}-A^{mls}-C^{e2s}-G^{mls}-A^{mls-}G^{e2s}-G^{mlt}-H (DUX4-048);
   HO-G^{mls}-C^{e2s}-G^{mls}-A^{mls}-A^{e2s}-G^{mls}-G^{mls}-C^{e2s}-G^{mls}-A^{mls}-C^{e2s}-C^{mls}-C^{mls}-A^{e2s}-C^{mls}-G^{mls}-A^{e2s}-G^{mlt}-H (DUX4-049);
   HO-U^{mls}-G^{e2s}-U^{mls}-G^{mls}-G^{e2s}-G^{mls}-C^{mls}-G^{e2s}-A^{mls}-A^{mls}-G^{e2s}-G^{mls}-C^{mls}-G^{e2s}-A^{mls}-C^{mls}-C^{e2s}-C^{mlt}-H (DUX4-052);
   HO-Gmls-T^{e2s}-G^{mls-}U^{mls}-G^{e2s}-G^{mls}-G^{mls}-C^{e2s}-G^{mls}-A^{mls}-A^{e2s}-G^{mls}-G^{mls}-C^{e2s}-G^{mls}-A^{mls}-C^{e2s}-C^{mlt}-H (DUX4-053);
   HO-C^{e2s}-G^{mls}-A^{mls}-A^{e2s}-G^{mls}-G^{mls}-C^{e2s}-G^{mls}-A^{mls}-C^{mls}-C^{mls}-C^{mls}-A^{e2s}-C^{mls}-G^{mls}-A^{e2s}-G^{mls}-G^{mlt}-H (DUX4-48.7);
   HO-C^{m1s}-G^{m1s}-A^{m1s}-A^{e2s}-G^{m1s}-G^{m1s}-C^{m1s}-G^{m1s}-A^{m1s}-C^{m1s}-C^{m1s}-C^{e2s}-A^{m1s}-C^{e2s}-G^{m1s}-A^{e2s}-G^{m1s}-G^{mlt}-H (DUX4-48.10);
   HO-C^{mls}-G^{mls}-A^{e2s}-A^{mls}-G^{mls}-G^{mls}-C^{mls}-G^{mls}-A^{e2s}-C^{mls}-C^{e2s}-C^{mls}-A^{mls}-C^{mls}-G^{mls}-A^{e2s}-G^{mls}-G^{mlt}-H (DUX4-48.11)
   HO-C^{e2s}-G^{mls}-A^{e2s}-A^{mls}-G^{mls}-G^{mls}-C^{mls}-G^{mls}-A^{mls}-C^{mls}-C^{mls}-C^{mls}-A^{mls}-C^{e2s}-G^{mls}-A^{e2s}-G^{mls}-G^{mlt}-H (DUX4-48.12);
   HO-C^{mls}-G^{mls}-A^{mls}-A^{e2s}-G^{mls}-G^{mls}-C^{mls}-G^{mls}-A^{mls-}C^{e2s-}C^{mls}-C^{mls}-A^{mls}-C^{e2s}-G^{mls}-A^{e2s}-G^{mls}-G^{mlt}-H (DUX4-48.14);
   HO-C^{mls}-G^{mls}-A^{mls}-A^{e2s}-G^{mls}-G^{mls}-C^{mls}-G^{mls}-A^{e2s}-C^{mls}-C^{mls}-C^{mls}-A^{mls}-C^{e2s}-G^{mls}-A^{e2s}-G^{mls}-G^{mlt}-H (DUX4-48.15);
   HO-C^{mls}-G^{mls}-A^{e2s}-A^{mls}-G^{mls}-G^{mls}-C^{e2s}-G^{mls}-A^{mls}-C^{e2s}-C^{mls}-C^{mls}-A^{mls}-C^{mls}-G^{mls}-A^{e2s}-G^{mls}-G^{mlt}-H (DUX4-48.19);
   HO-C^{mls}-G^{mls}-A^{mls}-A^{e2s}-G^{mls}-G^{mls}-C^{mls}-G^{mls}-A^{mls}-C^{mls}-C^{mls}-C^{mls}-A^{mls}-C^{e2s}-G^{mls}-A^{e2s}-G^{mls}-G^{mlt}-H (DUX4-48.20);
   HD-T^{e2s}-G^{mls}-T^{e2s}-G^{mls}-G^{mls}-G^{mls}-C^{e2s}-G^{mls}-A^{mls}-A^{mls}-G^{mls}-G^{mls}-C^{e2s}-G^{mls}-A^{mls}-C^{e2s}-C^{mls}-C^{e2t}-H (DUX4-52.1);
   HD-T^{e2s}-G^{mls}-T^{e2s}-G^{mls}-G^{mls}-G^{mls}-C^{e2s-}-G^{mls}-A^{mls-}A^{e2s}-G^{mls}-G^{mls}-C^{e2s}-G^{mls-}A^{mls}-C^{e2s}-C^{mls}-C^{mlt}-H (DUX4-52.2);
   HO-T^{e2s}-G^{mls}-U^{mls}-G^{mls}-G^{mls}-G^{mls}-C^{mls}-G^{mls}-A^{mls}-A^{mls}-G^{mls}-G^{mls}-C^{e2s}-G^{mls}-A^{mls}-C^{e2s}-C^{mls}-C^{e2t}-H (DUX4-52.7);
   HO-U^{mls}-G^{mls}-T^{e2s}-G^{mls}-G^{mls}-G^{mls}-C^{mls}-G^{mls}-A^{mls}-A^{mls}-G^{mls}-G^{mls}-C^{e2s}-G^{mls}-A^{e2s}-C^{mls}-C^{e2s}-C^{mlt}-H (DUX4-52.9)
   [wherein A^{e2s}, G^{e2s}, C^{e2s} and T^{e2s} represent corresponding ENAs (where the nucleobase of C is 5-methylcytosine) binding to a structure adjacent to the 3' side by a phosphorothioate bond; A^{mls}, G^{mls}, C^{mls} and U^{mls} represent corresponding 2'-OMe-RNAs binding to a structure adjacent to the 3' side by a phosphorothioate bond; C^{e2t} represents corresponding ENA (where the nucleobase of C is 5-methylcytosine) binding to a structure adjacent to the 3' side by a phosphodiester bond; and A^{mlt}, G^{mlt} and C^{mlt} represent corresponding 2-OMe-RNAs binding to a structure adjacent to the 3' side by a phosphodiester bond].
(13) The oligonucleotide or a pharmaceutically acceptable salt thereof of any one of (1) to (12) above, wherein an aminoalkylphosphate group containing a fatty acid(s) is further bound at the 5' or 3' end of the oligonucleotide.
(14) The oligonucleotide or a pharmaceutically acceptable salt thereof of (13) above, wherein the fatty acid is at least one selected from the group consisting of myristic acid, palmitic acid, stearic acid, arachidic acid and behenic acid.
(15) The oligonucleotide or a pharmaceutically acceptable salt thereof of any one of (1) to (14) above, for use in treatment of a disease or a symptom attributable to DUX4-fl expression.
(16) The oligonucleotide or a pharmaceutically acceptable salt thereof of (15) above, wherein the disease or symptom attributable to DUX4-fl expression is facioscapulohumeral muscular dystrophy.
(17) A pharmaceutical drug comprising the oligonucleotide or a pharmaceutically acceptable salt thereof of any one of (1) to (16) above.
(18) A therapeutic for a disease or a symptom attributable to DUX4-fl expression, which comprises the oligonucleotide or a pharmaceutically acceptable salt thereof of any one of (1) to (16) above.
(19) The therapeutic of (18) above, wherein the disease or symptom attributable to DUX4-fl expression is facioscapulohumeral muscular dystrophy.
(20) An agent capable of switching the splice form of the DUX4 gene from DUX4-fl to DUX4-s, which comprises the oligonucleotide or a pharmaceutically acceptable salt thereof of any one of (1) to (16) above.
(21) A method of treating a disease or a symptom attributable to DUX4-fl expression in a subject, comprising administering to the subject the oligonucleotide or a pharmaceutically acceptable salt thereof of any one of (1) to (16) above.
(22) The method of (21) above, wherein the disease or symptom attributable to DUX4-fl expression is facioscapulohumeral muscular dystrophy.
(23) Use of the oligonucleotide or a pharmaceutically acceptable salt thereof of any one of (1) to (16) above, for manufacturing a therapeutic for a disease or a symptom attributable to DUX4-fl expression.
(24) The use of (23) above, wherein the disease or symptom attributable to DUX4-fl expression is facioscapulohumeral muscular dystrophy.

### EFFECT OF THE INVENTION

According to the present invention, it becomes possible to reduce the transcription level of DUX4-fl by using an antisense oligonucleotide to switch the splice form of the DUX4 gene from DUX4-fl to DUX4-s.

The present specification encompasses the contents disclosed in the specification and/or the drawings of Japanese Patent Application No. 2019-129735 based on which the present patent application claims priority.

### BRIEF DESCRIPTION OF THE DRAWINGS

[Fig. 1] A diagram showing a method of using an antisense oligonucleotide to switch the splice form of the DUX4 gene from DUX4-fl to DUX4-s.
[Fig. 2] Gel-electrophoretic analysis of DUX4 transcription products obtained by transfecting the DUX4 minigene construct and compounds of Examples (DUX4-004 to DUX4-010) into HeLa cells.
[Fig. 3] Gel-electrophoretic analysis of DUX4 transcription products obtained by transfecting the DUX4 minigene construct and the compounds of Examples (DUX4-011 to DUX4-016) into HeLa cells.
[Fig. 4] Gel-electrophoretic analysis of DUX4 transcription products obtained by transfecting the DUX4 minigene construct and the compounds of Examples (DUX4-022 to DUX4-030).
[Fig. 5] Gel-electrophoretic analysis of DUX4 transcription products obtained by transfecting the DUX4 minigene construct and the compounds of Examples (DUX4-031 to DUX4-040) into HeLa cells.
[Fig. 6] Gel-electrophoretic analysis of DUX4 transcription products obtained by transfecting the DUX4 minigene construct and the compounds of Examples (DUX4-041 to DUX4-050) into HeLa cells.
[Fig. 7] Gel-electrophoretic analysis of DUX4 transcription products obtained by transfecting the DUX4 minigene construct and the compounds of Examples (DUX4-051 to DUX4-058) into HeLa cells.

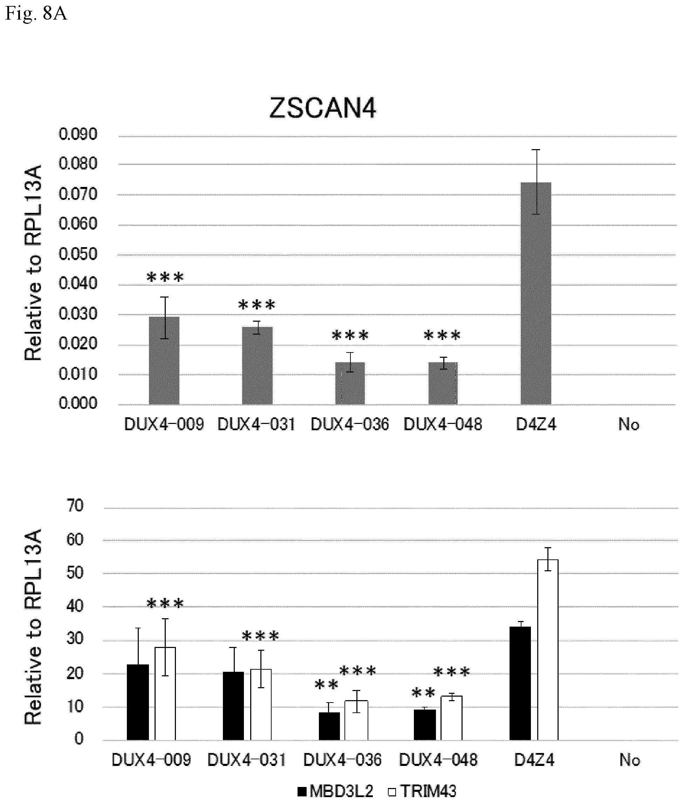

[Fig. 8A] Graphs showing the expression levels of the target genes (upper graph: ZSCAN4; lower graph: MBD3L2 and TRIM43) whose transcription are activated by DUX4. The vertical axis shows expression levels of the target genes relative to that of the RPL13A gene, an internal standard. In these graphs, "No" represents that cells were not transfected with either the DUX4 minigene construct or the compound of Examples (DUX4-009, DUX4-031, DUX4-036 or DUX4-048); "D4Z4" represents that cells were transfected with the DUX4 minigene construct alone; "DUX4-009", "DUX4-031", "DUX4-036" and "DUX4-048" represent that cells were transfected together with the DUX4 minigene and the compound of Examples (DUX4-009, DUX4-031, DUX4-036 or DUX4-048). Expession levels of the target gene tested in these cells are shown relative to that of RPL13A. Experiments were conducted in triplicates (N=3). For statistical analysis of the results, Dunnett test comparing with D4Z4 was used. Mark "^{∗}" indicates p<0.05; "^{∗∗}" indicates p<0.01; and "^{∗∗∗}" indicates p<0.001.

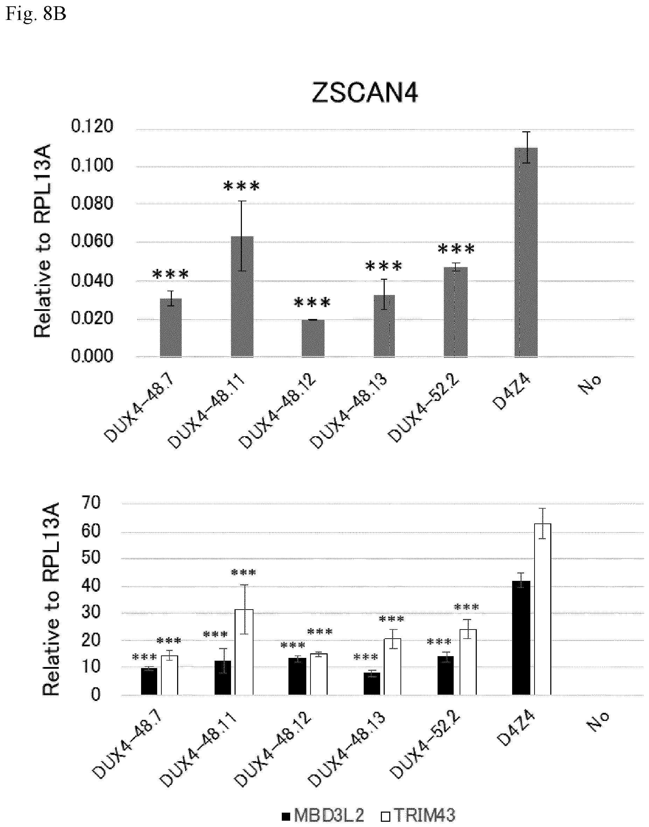

[Fig. 8B] Graphs showing the expression levels of the target genes (upper graph: ZSCAN4; lower graph: MBD3L2 and TRIM43) whose transcription are activated by DUX4. The vertical axis shows expression levels of the target genes relative to that of the RPL13A gene, an internal standard. In these graphs, "No" represents that cells were not transfected with either the DUX4 minigene construct or the compound of Examples (DUX4-48.7, DUX4-48.11, DUX4-48.12, DUX4-48.13, or DUX4-52.2); "D4Z4" represents that cells were transfected with the DUX4 minigene construct alone; "DUX4-48.7", "DUX4-48.11", "DUX4-48.12", " DUX4-48.13" and "DUX4-52.2" represent that cells were transfected with the DUX4 minigene and the compound of Examples (DUX4-48.7, DUX4-48.11, DUX4-48.12, DUX4-48.13 or DUX4-52.2). Expession levels of the target gene tested in these cells are shown relative to that of the RPL13A gene. Experiments were conducted in triplicates (N=3). For statistical analysis of the results, Dunnett test comparing with D4Z4 was used. Mark "^{∗}" indicates p<0.05; "^{∗∗}" indicates p<0.01; and "^{∗∗∗}" indicates p<0.001.

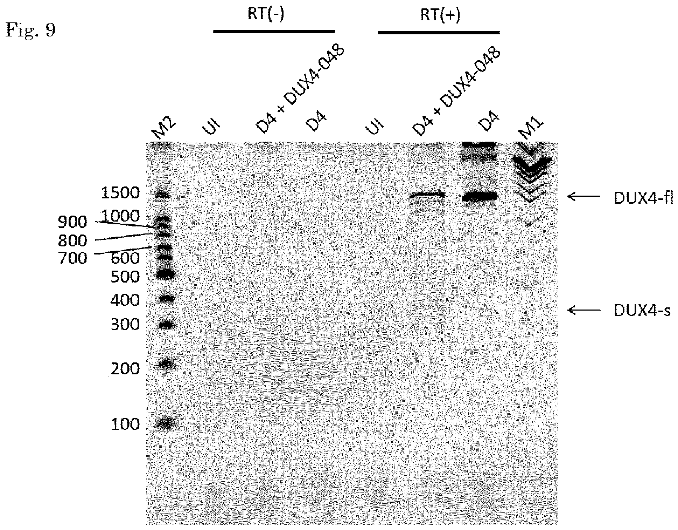

[Fig. 9] A diagram showing PCR products of D4Z4 mRNA-injected fertilized eggs of zebrafish. "RT(-)" represents the results of PCR using the template without reverse transcription, and "RT(+)" the results of PCR using the template with reverse transcription. "D4" represents that fertilized eggs were injected with the DUX4 minigene construct alone; and "D4 + DUX4-048" represents that fertilized eggs were injected with the both DUX4 minigene construct and the compound of the Example (DUX4-048). "M1" and "M2" represent molecular size markers, and "UI" represents PCR products amplified from uninjected fertilized eggs .
[Fig. 10] Gel-electrophoretic analysis of DUX4 transcription products obtained by transfecting the DUX4 minigene construct and the compounds of Examples (DUX4-48.1 to DUX4-48.10) into HeLa cells.
[Fig. 11] Gel-electrophoretic analysis of DUX4 transcription products obtained by transfecting the DUX4 minigene construct and the compounds of Examples (DUX4-48.11 to DUX4-48.13; DUX4-52.1 and DUX4-52.2) into HeLa cells.
[Fig. 12] Gel-electrophoretic analysis of DUX4 transcription products obtained by transfecting the DUX4 minigene construct and the compounds of Examples (DUX4-48.14 to DUX4-48.23; and DUX4-048) into HeLa cells.
[Fig. 13] Gel-electrophoretic analysis of DUX4 transcription products obtained by transfecting the DUX4 minigene construct and the compounds of Examples (DUX4-52.3 to DUX4-52.12; and DUX4-052) into HeLa cells.

### BEST MODES FOR CARRYING OUT THE INVENTION

Hereinbelow, embodiments of the present invention will be described in detail.

The present invention provides an oligonucleotide or a pharmaceutically acceptable salt or solvate thereof, wherein the oligonucleotide comprises an oligonucleotide of 15-30 bases consisting of a nucleotide sequence complementary to the region of nucleotide Nos. 502-556 or 578-612 (preferably, Nos. 506-549) of DUX4-fl mRNA consisting of the nucleotide sequence as shown in SEQ ID NO: 1; the 5' and/or 3' end of the oligonucleotide may be chemically modified; and the oligonucleotide is capable of switching the splice form of the DUX4 gene from DUX4-fl to DUX4-s.

The sequence information of the DUX4-fl mRNA is registered at the GenBank under the accession number of HQ266761. The nucleotide sequence of the DUX4-fl mRNA is shown in SEQ ID NO: 1 in the SEQUENCE LISTING. The sequence of the DUX4-s mRNA is the sequence of nucleotide Nos. 1-477 of the nucleotide sequence as shown in SEQ ID NO: 1.

DUX4-fl and DUX4-s are different splicing isoforms transcribed from DUX4 mRNA. DUX4-fl is a full-length type isoform (about 55 kDa) and DUX4-s is a short type isoform (about 20 kDa). DUX4-fl exerts a strong transcription activity via its C-terminal transcriptional activation domain, and it is considered that DUX4-fl is involved in the onset of FSHD. On the other hand, the short type DUX4-s does not function as a transcription factor because it does not have a transcriptional activation domain, though its expression is slightly observed even in cells from healthy individuals.

The oligonucleotide of the present invention is capable of switching the splice form of the DUX4 gene from DUX4-fl to DUX4-s. By switching the splice form of the DUX4 gene from DUX4-fl to DUX4-s, relative levels of DUX4-fl are reduced to thereby enable inhibition of cell death. Thus, therapeutic effect of the oligonuclrotides of the present invention for FSHD can be expected. Although DUX4-fl usually does not contain introns, some patients express DUX4-fl with intron 1 remained. Therefore, DUX4-fl may be either one with intron or without intron. The extent of switching the splice form of the DUX4 gene from DUX4-fl to DUX4-s is not particularly limited, but the switching rate is preferably 10% or more, and more preferably 25% or more. It is possible to measure the extent of switching the splice form of the DUX4 gene from DUX4-fl to DUX4-s by, for example, the method described below. Briefly, the oligonucleotide of the present invention is transfected into cells; total RNA is extracted from the transfected cells; after reverse transcription, double-stranded DNA is prepared by PCR using primers that amplify both DUX4-fl and DUX4-s. This double-stranded DNA thus amplified is subjected to gel electrophoresis to thereby separate the PCR products into two bands. By visualizing these two bands, it is possible to measure the extent of splice switching. When visualization of these bands is performed with ethidium bromide, SYBR Green or the like, determination of the extent of splice switching must be made carefully because the extent of visualization varies depending on the size of the amplified double-stranded DNA. Alternatively, it is also possible to measure the extent of splice switching as described below. Briefly, TaqMan proves specific for DUX4-fl and DUX4-s mRNAs are prepared and quantitative PCR is conducted with these probes, whereby expression levels of DUX4-fl and DUX4-s are quantified. The DUX4-fl and DUX4-s proteins in samples may be detected by Western blotting, or peptide fragments derived from the DUX4-fl and DUX4-s proteins may be detected separately by mass spectrometry, whereby the extent of splice switching can be measured.

As specific examples of the oligonucleotide of the present invention, oligonucleotides comprising a sequence of at least 15 consecutive nucleotides in any one of the sequences as shown in SEQ ID NOS: 2-85 (preferably, SEQ ID NOS: 5-31) (wherein "t" may be "u", and "u" may be "t") may be enumerated.

The number of bases of the oligonucleotide of the present invention is suitably 15-30, preferably 16-18, and more preferably 18.

As a preferable oligonucleotide of the present invention, an oligonucleotide of 15-30 bases may be given which consists of a nucleotide sequence complementary to the region of nucleotide Nos. 506-546 of the nucleotide sequence as shown in SEQ ID NO: 1. As a more preferable oligonucleotide of the present invention, an oligonucleotide may be given which comprises a sequence of at least 15 consecutive nucleotides in any one of the sequences as shown in SEQ ID NOS: 5-31 (wherein "t" may be "u", and "u" may be "t").

The oligonucleotide (antisense oligonucleotide) of the present invention may be either natural DNA, natural RNA, chimera DNA/RNA, or modified DNA, RNA or DNA/RNA. Preferably, at least one of the nucleotides constituting the oligonucleotide is a modified nucleotide.

Examples of modified nucleotides of the present invention include those in which a sugar is modified (e.g., the hydroxy group at 2'-position of D-ribofuranose is modified (D-ribofuranose is 2'-O-alkylated or D-ribofuranose is 2'-,4'-bridged (e.g., D-ribofuranose is 2'-O,4'-C-alkylenated), etc.), those in which a phosphodiester bond is modified (e.g. thioated), those in which a base is modified, combinations of the above-described nucleotides, and so forth. Antisense oligonucleotides in which at least one D-ribofuranose constituting the oligonucleotides is 2'-O-alkylated (e.g. 2'-O-methylated) or 2'-O,4'-C-alkylenated (e.g. 2'-O,4'-C-ethylenated) have high RNA binding strength and high resistance to nuclease. Thus, they are expected to produce higher therapeutic effect than natural nucleotides (i.e. oligo DNA or oligo RNA). Further, oligonucleotides in which at least one phosphodiester bond constituting the oligonucleotides is thioated also have high resistance to nuclease and, thus, are expected to produce higher therapeutic effect than natural nucleotides (i.e. oligo DNA or oligo RNA). Oligonucleotides comprising both the modified sugar and the modified phosphate as described above have even higher resistance to nuclease and, thus, are expected to produce even higher therapeutic effect.

With respect to the oligonucleotide (antisense oligonucleotide) of the present invention, examples of modified sugars include, but are not limited to, D-ribofuranose as 2'-O-alkylated (e.g. 2'-O-methylated, 2'-O-aminoethylated, 2'-O-propylated, 2'-O-allylated, 2'-O-methoxyethylated, 2'-O-butylated, 2'-O-pentylated, or 2'-O-propargylated); D-ribofuranose as 2'-O,4'-C-alkylenated (e.g. 2'-O,4'-C-ethylenated, 2'-O,4'-C-methylenated, 2'-O,4'-C-propylenated, 2'-O,4'-C-tetramethylenated, or 2'-O,4'-C-pentamethylenated); D-ribofuranose as 2'-,4'-bridged, for example, S-cEt (2',4'-constrained ethyl), AmNA (Amidebridged nucleic acid), etc.; D-ribofuranose as 2'-deoxy-2'-C,4'-C-methyleneoxymethylated, or D-ribofuranose as 2' deoxygenated in combination with other modifications, for example, 3'-deoxy-3'-amino-2'-deoxy-D-ribofuranose, 3'-deoxy-3'-amino-2'-deoxy-2'-fluoro-D-ribofuranose, etc.

With respect to the oligonucleotide (antisense oligonucleotide) of the present invention, examples of the modification of phosphodiester bond include, but are not limited to, phosphorothioate bond, methylphosphonate bond, methylthiophosphonate bond, phosphorodithioate bond and phosphoroamidate bond.

With respect to the oligonucleotide (antisense oligonucleotide) of the present invention, examples of modified bases include, but are not limited to, cytosine as 5-methylated, 5-fluorinated, 5-brominated, 5-iodinated or N4-methylated; thymine as 5-demethylated (uracil), 5-fluorinated, 5-brominated or 5-iodinated; adenine as N6-methylated or 8-brominated; and guanine as N2-methylated or 8-brominated.

Nucleotide residues constituting the oligonucleotide of the present invention include A^{t},G^{t}, 5meC^{t}, C^{t}, T^{t}, U^{t}, A^{p}, G^{p}, 5meC^{p}, C^{p}, T^{p}, U^{p}, A^{s}, G^{s}, 5meC^{s}, C^{s}, T^{s}, U^{s}, A^{mlt}, G^{mlt}, C^{mlt}, 5meC^{mlt}, U^{mlt}, A^{mlp}, G^{mlp}, C^{mlp}, 5meC^{mlp}, U^{mlp}, A^{mls}, C^{mls}, C^{mls}, 5meC^{mls}, U^{mls}, A^{2t}, G^{2t}, C^{2t}, T^{2t}, A^{e2p}, G^{e2p}, C^{e2p}, T^{e2p}, A^{e2s}, G^{e2s}, C^{e2s}, T^{e2s}, A^{lt}, G^{lt}, C^{lt}, T^{lt}, A^{elp}, G^{elp}, C^{elp}, T^{elp}, A^{els}, G^{els}, C^{els}, T^{els}, A^{3t}, G^{3t}, C^{3t}, T^{3t}, A^{e3p}, G^{e3p}, C^{e3p}, T^{e3p}, A^{e3s}, G^{e3s}, C^{e3s}, T^{e3s}, A^{m2t} G^{m2t}, 5meC^{m2t}, T^{m2t}, A^{m2p}, G^{m2p}, 5meC^{m2p}, T^{m2p}, A^{m2s}, G^{m2s}, 5meC^{m2s}, and T^{m2s}, the respective structures of which are shown in one Example described later.

As the oligonucleotide of the present invention, preferable examples are shown in Tables 1 to 3, and more preferable examples are shown below.
HO-G^{m1s}-G^{e2s}-G^{m1s}-A^{m1s}-G^{e2s}-C^{m1s}-A^{m1s}-G^{e2s}-G^{m1s}-G^{m1s}-T^{e2s}-G^{m1s}-A^{m1s}-C^{e2s}-C^{m1s}-C^{m1s}-C^{e2s}-C^{mlt}-H (DUX4-006);
HD-G^{mls}-A^{e2s}-C^{mls}-C^{mls}-C^{e2s}-A^{mls}-C^{mls}-G^{e2s}-A^{mls}-G^{mls}-G^{e2s}-G^{mls}-A^{mls}-G^{e2s}-C^{mls}-A^{mls}-G^{e2s}-G^{mlt}-H (DUX4-009);
HO-G^{mls}-A^{e2s}-A^{mls}-G^{mls}-G^{e2s}-C^{mls}-G^{mls}-A^{e2s}-C^{mls}-C^{mls-}C^{e2s}-A^{mls}-C^{mls}-G^{e2s}-A^{mls}-G^{mls}-G^{e2s}-G^{mlt}-H (DUX4-011);
HO-G^{m1s}-G^{e2s}-U^{m1s}-G^{m1s}-T^{e2s}-G^{m1s}-G^{m1s}-G^{e2s}-C^{m1s}-G^{m1s}-A^{e2s}-A^{m1s}-G^{m1s}-G^{e2s}-C^{m1s}-G^{m1s}-A^{e2s}-C^{mlt}-H (DUX4-014);
HO-G^{m1s}-A^{e2s}-G^{m1s}-C^{m1s}-A^{e2s}-G^{m1s}-G^{m1s}-G^{e2s}-U^{m1s}-G^{m1s}-A^{e2s}-C^{m1s}-C^{m1s}-C^{e2s}-C^{m1s}-C^{m1s}-G^{e2s}-C^{mlt}-H (DUX4-036);
HO-G^{m1s}-G^{e2s}-A^{m1s}-G^{m1s}-C^{e2s}-A^{m1s}-G^{m1s}-G^{e2s}-G^{m1s}-U^{m1s}G^{e2s}-A^{m1s}-C^{m1s}-C^{e2s}-C^{m1s}-C^{m1s}-C^{e2s}-G^{m1t}-H (DUX4-037);
HO-A^{m1s}-C^{e2s}-G^{m1s}-A^{m1s}-G^{e2s}-G^{m1s}-G^{m1s}-A^{e2s}-G^{m1s}-C^{m1s}-A^{e2s}-G^{m1s}-G^{m1s}-G^{e2s}-U^{m1s}-G^{m1s}-A^{e2s}-C^{mlt}-H (DUX4-040);
HO-C^{m1s}-G^{e2s}-A^{m1s}-C^{m1s}-C^{e2s}-C^{m1s}-A^{m1s}-C^{e2s}-G^{m1s}-A^{m1s}-G^{e2s}-G^{m1s}-G^{m1s}-A^{e2s}-G^{m1s}-C^{m1s}-A^{e2s}-G^{mlt}-H (DUX4-044);
HO-A^{m1s}-A^{e2s}-G^{m1s}-G^{m1s}-C^{e2s}-G^{m1s}-A^{m1s}-C^{e2s}-C^{m1s}-C^{m1s}-A^{e2s}-C^{m1s}-G^{m1s}-A^{e2s}-G^{m1s}-G^{m1s}-G^{e2s}-A^{mlt}-H (DUX4-047);
HO-C^{m1s}-G^{e2s}-A^{m1s}-A^{m1s}-G^{e2s}-G^{m1s}-C^{m1s}-G^{e2s}-A^{m1s}-C^{m1s}-C^{e2s}-C^{m1s}-A^{m1s}-C^{e2s}-G^{m1s}-A^{m1s}-G^{e2s}-G^{mlt}-H (DUX4-048);
HO-G^{m1s}-C^{e2s}-G^{m1s}-A^{m1s}-A^{e2s}-G^{m1s}-G^{m1s}-C^{e2s}-G^{m1s}-A^{m1s}-C^{e2s}-C^{m1s}-C^{m1s}-A^{e2s}-C^{m1s}-G^{m1s}-A^{e2s}-G^{mlt}-H (DUX4-049);
HO-U^{m1s}-G^{e2s}-U^{m1s}-G^{m1s}-G^{e2s}-G^{m1s}-C^{m1s}-G^{e2s}-A^{m1s}-A^{m1s}-G^{e2s}-G^{m1s}-C^{m1s}-G^{e2s}-A^{m1s}-C^{m1s}-C^{e2s}-C^{mlt}-H (DUX4-052);
HO-G^{m1s}-T^{e2s}-G^{m1s}-U^{m1s}-G^{e2s}-G^{m1s}-G^{m1s}-C^{e2s}-G^{m1s}-A^{m1s}-A^{e2s}-G^{m1s}-G^{m1s}-C^{e2s}-G^{m1s}-A^{m1s}-C^{e2s}-C^{mlt}-H (DUX4-053);
HO-C^{e2s}-G^{m1s}-A^{m1s}-A^{e2s}-G^{m1s}-G^{m1s}-C^{e2s}-G^{m1s}-A^{m1s}-C^{m1s}-C^{m1s}-C^{m1s}-A^{e2s}-C^{m1s}-G^{m1s}-A^{e2s}-G^{m1s}-G^{mlt}-H (DUX4-48.7);
HO-C^{m1s}-G^{m1s}-A^{m1s}-A^{e2s}-G^{m1s}-G^{m1s}-C^{m1s}-G^{m1s}-A^{m1s}-C^{m1s}-C^{m1s}-C^{e2s}-A^{m1s}-C^{e2s}-G^{m1s}-A^{e2s}-G^{m1s}-G^{mlt}-H (DUX4-48.10);
HO-C^{m1s}-G^{m1s}-A^{e2s}-A^{m1s}-G^{m1s}-G^{m1s}-C^{m1s}-G^{m1s}-A^{e2s}-C^{m1s}-C^{e2s}-C^{m1s}-A^{m1s}-C^{m1s}-G^{m1s}-A^{e2s}-G^{m1s}-G^{mlt}-H (DUX4-48.11);
HO-C^{e2s}-G^{m1s}-A^{e2s}-A^{m1s}-G^{m1s}-G^{m1s}-C^{m1s}-G^{m1s}-A^{m1s}-C^{m1s}-C^{m1s}-C^{m1s}-A^{m1s}-C^{e2s}-G^{m1s}-A^{e2s}-G^{m1s}-G^{mlt}-H (DUX4-48.12);
HO-C^{m1s}-G^{m1s}-A^{m1s}-A^{e2s}-G^{m1s}-G^{m1s}-C^{m1s}-G^{m1s}-A^{m1s}-C^{e2s}-C^{m1s}-C^{m1s}-A^{m1s}-C^{e2s}-G^{m1s}-A^{e2s}-G^{m1s}-G^{mlt}-H (DUX4-48.14);
HO-C^{m1s}-G^{m1s}-A^{m1s}-A^{e2s}-G^{m1s}-G^{m1s}-C^{m1s}-G^{m1s}-A^{e2s}-C^{m1s}-C^{m1s}-C^{m1s}-A^{m1s}-C^{e2s}-G^{m1s}-A^{e2s}-G^{m1s}-G^{mlt}-H (DUX4-48.15);
HO-C^{m1s}-G^{m1s}-A^{e2s}-A^{m1s}-G^{m1s}-G^{m1s}-C^{e2s}-G^{m1s}-A^{m1s}-C^{e2s}-C^{m1s}-C^{m1s}-A^{m1s}-C^{m1s}-G^{m1s}-A^{e2s}-G^{m1s}-G^{mlt}-H (DUX4-48.19);
HO-C^{m1s}-G^{m1s}-A^{m1s}-A^{e2s}-G^{m1s}-G^{m1s}-C^{m1s}-G^{m1s}-A^{m1s}-C^{m1s}-C^{m1s}-C^{m1s}-A^{m1s}-C^{e2s}-G^{m1s}-A^{e2s}-G^{m1s}-G^{mlt}-H (DUX4-48.20);
HO-T^{e2s}-G^{m1s}-T^{e2s}-G^{m1s}-G^{m1s}-G^{m1s}-C^{e2s}-G^{m1s}-A^{m1s}-A^{m1s}-G^{m1s}-G^{m1s}-C^{e2s}-G^{m1s}-A^{m1s}-C^{e2s}-C^{m1s}-C^{e2t}-H (DUX4-52.1);
HO-T^{e2s}-G^{m1s}-T^{e2s}-G^{m1s}-G^{m1s}-G^{m1s}-C^{e2s}-G^{m1s}-A^{m1s}-A^{e2s}-G^{m1s}-G^{m1s}-C^{e2s}-G^{m1s}-A^{m1s}-C^{e2s}-C^{m1s}-C^{mlt}-H (DUX4-52.2);
HO-T^{e2s}-G^{m1s}-U^{m1s}-G^{m1s}-G^{m1s}-G^{m1s}-C^{m1s}-G^{m1s}-A^{m1s}-A^{m1s}-G^{m1s}-G^{mls}-C^{e2s}-G^{m1s}-A^{m1s}-C^{e2s}-C^{m1s}-C^{e2t}-H (DUX4-52.7);
HO-U^{m1s}-G^{m1s}-T^{e2s}-G^{m1s}-G^{m1s}-G^{m1s}-C^{m1s}-G^{m1s}-A^{m1s}-A^{m1s}-G^{m1s}-G^{m1s}-C^{e2s}-G^{m1s}-A^{e2s}-C^{m1s}-C^{e2s}-C^{mlt}-H (DUX4-52.9)

The oligonucleotide (antisense oligonucleotide) of the present invention may be synthesized with a commercially available DNA synthesizer (e.g., PerkinElmer Model 392 based on the phosphoramidite method) according to the method described in Nucleic Acids Research, 12, 4539 (1984) with necessary modifications. As phosphoramidite reagents to be used in the process, natural nucleosides and 2'-O-methylnucleosides (i.e., 2'-O-methylguanosine, 2'-O-methyladenosine, 2'-O-methylcytidine and 2'-O-methyluridine) are commercially available. As regards 2'-O-alkylguanosine, -alkyladenosine, -alkylcytidine and -alkyluridine in which the carbon number of the alkyl group is 2-6, the following methods may be employed.

2'-O-aminoethylguanosine, 2'-O-aminoethyladenosine, 2'-O-aminoethylcytidine and 2'-O-aminoethyluridine may be synthesized as previously described (Blommers et al., Biochemistry (1998), 37, 17714-17725).

2'-O-propylguanosine, 2'-O-propyladenosine, 2'-O-propylcytidine and 2'-O-propyluridine may be synthesized as previously described (Lesnik, E.A. et al., Biochemistry (1993), 32, 7832-7838).

2'-O-allylguanosine, 2'-O-allyladenosine, 2'-O-allylcytidine and 2'-O-allyluridine may be commercially available as reagents.

2'-O-methoxyethylguanosine, 2'-O-methoxyethyladenosine, 2'-O-methoxyethylcytidine and 2'-O-methoxyethyluridine may be synthesized as previously described (US Patent No. 6261840 or Martin, P. Helv. Chim. Acta. (1995) 78, 486-504).

2'-O-butylguanosine, 2'-O-butyladenosine, 2'-O-butylcytidine and 2'-O-butyluridine may be synthesized as previously described (Lesnik, E.A. et al., Biochemistry (1993), 32, 7832-7838).

2'-O-pentylguanosine, 2'-O-pentyladenosine, 2'-O-pentylcytidine and 2'-O-pentyluridine may be synthesized as previously described (Lesnik, E.A. et al., Biochemistry (1993), 32, 7832-7838).

2'-O-propargylguanosine, 2'-O-propargyladenosine, 2'-O-propargylcytidine and 2'-O -propargyluridine may be commercially available as reagents.

2'-O,4'-C-methyleneguanosine, 2'-O,4'-C-methyleneadenosine, 2'-O,4'-C-methylenecytidine, 2'-O,4'-C-methylene-5-methylcytidine and 2'-O,4'-C-methylenethymidine may be prepared according to the method described in WO99/14226; and 2'-O,4'-C-alkyleneguanosine, 2'-O,4'-C-alkyleneadenosine, 2'-O,4'-C-methylenecytidine, 2'-O,4'-C-alkylene-5-methylcytidine and 2'-O,4'-C-alkylenethymidine in which the carbon number of the alkylene group is 2-5 may be prepared according to the method described in WO00/47599.

Nucleosides in which D-ribofuranose is 2'-deoxy-2'-C,4'-C-methyleneoxymethylenated may be synthesized as previously described (Wang, G. et al., Tetrahedron (1999), 55, 7707-7724).

S-cEt (constrained ethyl) may be synthesized as previously described (Seth, P.P. et al. J. Org. Chem (2010), 75, 1569-1581).

AmNA may be synthesized as previously described (Yahara, A. et al. ChemBioChem (2012), 13, 2513-2516; or WO2014/109384).

In the present invention, nucleobase sequences may be described using the abbreviation (A) or (a) for adenine, (G) or (g) for guanine, (C) or (c) for cytosine, (T) or (t) for thymine, and (U) or (u) for uracil. Instead of cytosine, 5-methylcytosine may be used. Among the nucleobases, uracil (U) or (u) and thymine (T) or (t) are interchangeable. Both uracil (U) or (u) and thymine (T) or (t) may be used in base pairing with adenine (A) or (a) in the complementary strand.

An antisense oligonucleotide with phophorothioate bonds can be synthesized by coupling phosphoramidite reagents and then reacting sulfur, tetraethylthiuram disulfide (TETD; Applied Biosystems), Beaucage reagent (Glen Research) or a reagent such as xanthan hydride (Tetrahedron Letters, 32, 3005 (1991); J. Am. Chem. Soc. 112, 1253 (1990); PCT/WO98/54198).

As controlled pore glass (CPG) to be used in a DNA synthesizer, 2'-O-methylnucleoside-bound CPG is commercially available. As regards 2'-O,4'-C-methyleneguanosine, 2'-O,4'-C-methyleneadenosine, 5-methylcytidine and thymidine, they may be prepared according to the method described in WO99/14226; and as regards 2'-O,4'-C-alkyleneguanosine, adenosine, 5-methylcytidine and thymidine in which the carbon number of the alkylene group is 2-5, they may be prepared according to the method described in WO00/47599. The nucleotides thus prepared may then be bound to CPG as previously described (Oligonucleotide Synthesis, Edited by M. J. Gait, Oxford University Press, 1984). By using the modified CPG (as disclosed in Example 12b of Japanese Unexamined Patent Publication No. Hei7-87982), an oligonucleotide in which a 2-hydroxyethylphosphate group is bound at the 3' end can be synthesized. If 3'-amino-Modifier C3 CPG, 3'-amino-Modifier C7 CPG or Glyceryl CPG (Glen Research) or 3'-specer C3 SynBase CPG 1000 or 3'-specer C9 SynBase CPG 1000 (Link Technologies) is used, an oligonucleotide in which a hydroxyalkylphosphate group or aminoalkylphosphate group is bound at the 3' end can be synthesized.

The oligonucleotide of the present invention may be chemically modified by binding to its 3' and/or 5' end a molecular structure other than nucleotide via a phosphodiester bond or phosphorothioate bond. The present invention also provides such chemically modified oligonucleotides.

If 5'-Amino-Modifier C6 (Glen Research), 5'-TFA-Amino-Modifier C6-CE Phosphoramidite, 5'-TFA-Amino-Modifier-C5-CE Phosphoramidite (Link Technologies) or the like is used after completion of the chain elongation of an oligonucleotide having a sequence of interest, an oligonucleotide in which an aminoalkylphosphate group is bound at the 5' end can be synthesized.

The oligonucleotide (antisense oligonucleotide) of the present invention may have a hydrophobic group at the 5' and/or 3' end. A fatty acid-containing aminoalkylphosphate group may be further bound at the 5' and/or 3' end of the oligonucleotide (antisense oligonucleotide) of the present invention. For example, the oligonucleotide of the present invention may have, instead of a hydroxyl group, a group represented by one of the following formulas at the 5' and/or 3' end.

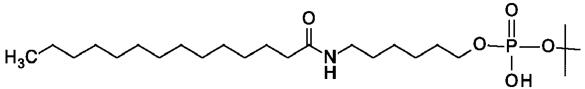

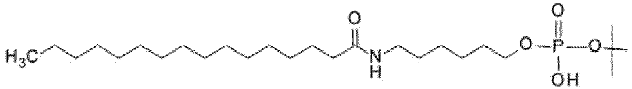

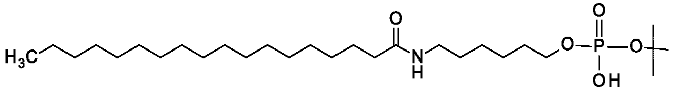

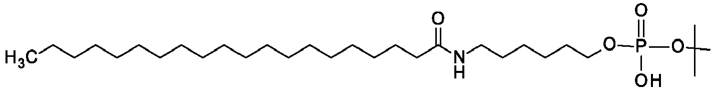

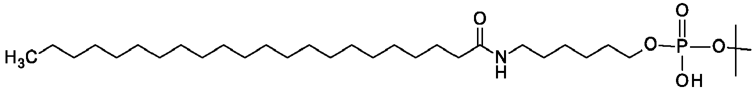

Such an oligonucleotide can be synthesized by coupling an amidite unit corresponding to a predetermined fatty acid such as 2-Cyanoethyl (6-palmitamidohexyl) diisopropylphosphoramidite (Nucleic Acids Res. (2020) 47, 6029-6044; Link Technologies) after completion of the chain elongation of an oligonucleotide having a sequence of interest.

The oligonucleotide as described above can be synthesized by reacting an oligonucleotide which has an aminoalkylphosphate group (e.g., with a carbon number of 3-9 as alkyl) at the 5' and/or 3' end and yet has a sequence of interest with an active ester such as pentafluorophenyl ester of fatty acid (e.g. myristic acid, palmitic acid, stearic acid, arachidic acid, behenic acid, etc.) (Nucleic Acids Res. (2020) 47, 6029-6044). Alternatively, it is also possible to synthesize the oligonucleotide as described above by the following method. In brief, at the time of solid phase synthesis of an oligonucleotide having a sequence of interest, the oligonucleotide is condensed with a fatty acid (such as myristic acid, palmitic acid, stearic acid, arachidic acid or behenic acid) on a solid-phase carrier having an aminoalkylphosphate group (e.g., with a carbon number of 3-9 as alkyl) at the 5' end using a condensation agent such as HATU; and then deprotected/purified (PCT/WO2017/192679).

Further, if 5'-Tocopherol-CE Phosphoramidite, 5'-Cholesterol-CE Phosphoramidite, 5'-Cholesterol-TEG-CE Phosphoramidite (Link Technologies) or the like is used, an oligonucleotide in which cholesterol or tocopherol is bound at the 5' end can be synthesized.

The oligonucleotide (antisense oligonucleotide) of the present invention may be used as a pharmaceutical drug, in particular, for treating facioscapulohumeral muscular dystrophy (FSHD). The treatment may be conducted either before the onset of the disease (prevention), simultaneously with the onset thereof, or after the onset thereof.

The oligonucleotide (antisense oligonucleotide) of the present invention may be used in the form of a pharmaceutically acceptable salt. The term "pharmaceutically acceptable salt" as used herein refers to salts of the oligonucleotide (antisense oligonucleotide). Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic amine salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; inorganic acid salts including hydrohalogenic acid salts such as hydrofluorides, hydrochlorides, hydrobromides or hydroiodides, as well as nitrates, perchlorates, sulfates or phosphates; organic acid salts including lower alkane sulfonic acid salts such as methanesulfonates, trifluoromethanesulfonates or ethanesulfonates, arylsulfonic acid salts such as benzenesulfonates or p-toluenesulfonates, as well as acetates, malates, fumarates, succinates, citrates, tartrates, oxalates or maleates; and amino acid salts such as glycine salts, lysine salts, arginine salts, ornithine salts, glutamic acid salts or aspartic acid salts. These salts may be prepared by known methods.

The oligonucleotide (antisense oligonucleotide) sometimes occur as a solvate (e.g. hydrate). Such a solvate is also encompassed in a pharmaceutically acceptable salt of the oligonucleotide of the present invention.

Therefore, the present invention provides a pharmaceutical drug, comprising the above-described oligonucleotide or a pharmaceutically acceptable salt thereof. The present invention also provides a therapeutic for a disease or a symptom attributable to DUX4-fl expression, which comprises the above-described oligonucleotide or a pharmaceutically acceptable salt thereof. In particular, the present invention provides a therapeutic for facioscapulohumeral muscular dystrophy.

The DUX4-fl protein activates the expression of genes such as PRAMEF2, TRIM43, ZSCAN4, etc. expressing in early embryo, testis, or the like (Non-Patent Document No. 19: Geng et al., Dev Cell 2012, 22:38-51). It has been reported that the expression of DUX4-fl causes apoptosis (Non-Patent Document No. 20: Kowaljow et al., Neuromuscul Disord 2007, 17:611-623; Non-Patent Document No. 21: Wallace et al., Ann Neurol 2011, 69:540-552), abnormal muscle development (Non-Patent Document No. 22: Bosnakovski et al., EMBO J 2008, 27:2766-2779; Non-Patent Document No. 23: Banerji et al., Nat Commun 2017, 8:2152), inhibition of nonsense-mediated mRNA decay (Non-Patent Document No. 24: Feng et al., eLife 2015, 4:e04996), formation of intracellular double-stranded RNA (Non-Patent Document No. 25: Shadle et al., PLoS Genet 2017, 13:e1006658), protein aggregation (Non-Patent Document No. 26: Homma et al., Ann Clin Transl Neurol 2015, 2:151-166) and so forth. Therefore, expression of DUX4-fl can be a cause of various diseases and symptoms. Since the oligonucleotide of the present invention switches the splicing of DUX4 mRNA from DUX4-fl with strong transcriptional activity to DUX4-s without transcriptional activity, the oligonucleotide has an effect of treating those diseases or symptoms attributable to the transcriptional activity of DUX4-fl.

The therapeutic for facioscapulohumeral muscular dystrophy of the present invention may be applied to patients expressing DUX4-fl. Various researches including the report by Lemmers et al. (Non-Patent Document No. 8: Lemmers et al., Science 2010, 329:1650-1653) have shown that DUX4-fl is expressed in patients with haplotype 4qA, regardless of FSHD1 or FSHD2 patients. Therefore, the present invention is applicable to both FSHD1 and FSHD2.

When the oligonucleotide (antisense oligonucleotide) of the present invention or a pharmaceutically acceptable salt thereof is used for treating facioscapulohumeral muscular dystrophy, they may be administered *per se* or mixed with appropriate, pharmaceutically acceptable excipients, diluents, and the like for oral administration in the form of tablets, capsules, granules, powders, syrups, etc. or for parenteral administration in the form of injections, suppositories, patches or external preparations.

These formulations may be prepared by well-known methods using additives such as excipients (e.g., organic excipients including sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; or pullulan; and inorganic excipients including silicate derivatives such as light anhydrous silicic acid, synthetic aluminum silicate, calcium silicate or magnesium aluminometasilicate; phosphates such as calcium hydrogenphosphate; carbonates such as calcium carbonate; and sulfates such as calcium sulfate), lubricants (e.g., stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate or magnesium lauryl sulfate; silicic acid compounds such as silicic anhydride and silicic hydrate; or the starch derivatives listed above), binders (e.g., hydroxypropylcellulose, hydroxypropylmethylcellulose, polyvinylpyrrolidone, macrogol, or compounds similar to the above-listed excipients), disintegrants (e.g., cellulose derivatives such as low-substituted hydroxypropylcellulose, carboxymethylcellulose, calcium carboxymethylcellulose or internally crosslinked sodium carboxymethylcellulose; and chemically modified starch/cellulose derivatives such as carboxymethylstarch, sodium carboxymethylstarch or crosslinked polyvinylpyrrolidone), emulsifiers (e.g., colloidal clay such as bentonite or veegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylenealkylether, polyoxyethylene sorbitan fatty acid ester or sucrose esters of fatty acids), stabilizers (e.g., p-hydroxybenzoate esters such as methylparaben or propylparaben; alcohols such as chlorobutanol, benzyl alcohol or phenyl ethyl alcohol; benzalkonium chloride; phenols such as phenol or cresol; thimerosal; dehydroacetic acid; or sorbic acid), flavoring agents (e.g., conventionally used sweeteners, acidifiers, flavors and the like) or diluents.

The therapeutic of the present invention may comprise 0.1-250 µmoles/ml, preferably 1-50 µmoles/ml of oligonucleotide (antisense oligonucleotide), or a pharmaceutically acceptable salt thereof; 0.02-10% w/v of carbohydrate or polyalcohol; and 0.01-0.4% w/v of pharmaceutically acceptable surfactant.

As the above carbohydrate, monosaccharides or disaccharides are especially preferable. Specific examples of these carbohydrates and polyalcohols include, but are not limited to, glucose, galactose, mannose, lactose, maltose, mannitol and sorbitol. These may be used alone or in combination.

Preferable examples of the surfactant include, but are not limited to, polyoxyethylene sorbitan mono-, di- or tri-ester, alkylphenylpolyoxyethylene, sodium taurocholate, sodium cholate and polyalcohol esters. Among these, polyoxyethylene sorbitan mono-, di- and tri-ester are especially preferable; the most preferable esters are oleate, laurate, stearate and palmitate. These may be used alone or in combination.

More preferably, the therapeutic of the present invention may comprise 0.03-0.09 M pharmaceutically acceptable neutral salt such as sodium chloride, potassium chloride and/or calcium chloride.

Even more preferably, the therapeutic of the present invention may comprise 0.002-0.05 M pharmaceutically acceptable buffer. Examples of a preferable buffer include, but are not limited to, sodium citrate, sodium glycinate, sodium phosphate and tris(hydroxymethyl)aminomethane. These buffers may be used alone or in combination.

Further, the above-described therapeutic may be supplied in a state of solution. However, as in the case where there is a need for storage over a certain period of time, the therapeutic is preferably lyophilized for stabilizing the oligonucleotide (antisense oligonucleotide) to thereby prevent the lowering of its therapeutic effect. When lyophilized, the therapeutic may be reconstructed with a solution, such as distilled water for injection, just before use. Thus, the therapeutic is returned into the state of a liquid to be administered. Therefore, the therapeutic of the present invention encompasses one in a lyophilized state that is used after reconstruction with a solution so that the respective components fall within specified concentration ranges. For the purpose of promoting the solubility of the lyophilized product, the therapeutic may further comprise albumin and amino acids such as glycine.

When the oligonucleotide (antisense oligonucleotide) of the invention or a pharmaceutically acceptable salt thereof is administered to a human, the oligonucleotide or the like may be administered, for example, at approximately 0.01-100 mg/kg (body weight), preferably at 0.1-20 mg/kg (body weight) per adult per day either once or over several times by subcutaneous injection, intravenous infusion or intravenous injection. The dose and the number of times of administration may be changed appropriately depending on the type and symptoms of the disease, the age of the patient, administration route, etc.

Administration of the oligonucleotide (antisense oligonucleotide) of the invention or a pharmaceutically acceptable salt thereof to human (e.g., patient expressing DUX4-fl) may be performed, for example, as described below. Briefly, the oligonucleotide (antisense oligonucleotide) or a pharmaceutically acceptable salt thereof is prepared by methods well-known to one of ordinary skill in the art and sterilized by conventional methods to prepare, for example, 125 mg/ml of an injection solution. This solution is instilled to a patient intravenously in the form of, for example, infusion so that the dose of the oligonucleotide (antisense oligonucleotide) is, for example, 10 mg per kg body weight. This administration is repeated, for example, at 1-week intervals. Subsequently, this treatment is appropriately repeated while confirming the therapeutic effect.

Further, the oligonucleotide (antisense oligonucleotide) of the invention or a pharmaceutically acceptable salt thereof may be used for switching the splice form of the DUX4 gene from DUX4-fl to DUX4-s. Therefore, the present invention provides an agent capable of switching the splice form of the DUX4 gene from DUX4-fl to DUX4-s, which comprises the above-described oligonucleotide or a pharmaceutically acceptable salt thereof. As an agent capable of switching the splice form of the DUX4 gene from DUX4-fl to DUX4-s, an oligonucleotide or a pharmaceutically acceptable salt thereof capable of increasing the expression level of DUX4-s in DUX4-fl-expressing cells by about 10% or more, preferably by 30% or more, more preferably by 50% or more, relative to the level in control cells may be used. However, as long as an oligonucleotide has the function of switching the transcription product from DUX4-fl to DUX4-s, the oligonucleotide or a pharmaceutically acceptable salt thereof may be used even if they do not satisfy the above-specified ranges. The agent of the present invention may be used as a pharmaceutical drug or a reagent for experiments.

When the agent of the present invention is used as a reagent for experiments, the splicing form of the DUX4 gene can be switched from DUX4-fl to DUX4-s by treating DUX4-fl-expressing cells, tissues or organs with the oligonucleotide (antisense oligonucleotide) of the present invention or a pharmaceutically acceptable salt thereof. The oligonucleotide (antisense oligonucleotide) of the present invention or a pharmaceutically acceptable salt thereof may be used in an amount effective for switching the splice form of the DUX4 gene from DUX4-fl to DUX4-s. The DUX4-fl-expressing cells may be exemplified by ES cells, iPS cells, and naturally occurring cells such as FSHD patient-derived myoblast. In addition to the naturally occurring cells, the DUX4 gene-tranfected recombinant cells may also be employed. The DUX4-fl-expressing tissues and organs may be exemplified by testis, early embryo at the four-cell stage, and skeletal muscle of FSHD patients. Expression of the DUX4-fl mRNA or protein may be analyzed by amplifying the DUX4-fl mRNA (transcription product) in samples by RT-PCR, by detecting the DUX4-fl protein (translation product) in samples by Western blotting, or by detecting DUX4-fl-derived peptide fragments by mass spectrometry.

### EXAMPLES

Hereinbelow, the present invention will be described in detail with reference to the following Examples. These Examples are given only for explanation purposes and are not intended to limit the scope of the present invention.

### [Example 1] Synthesis of HO-C^{m1s}-T^{e2s}-G^{m1s}-U^{m1s}-G^{e2s}-G^{m1s}-G^{m1s}-A^{e2s}-G^{m1s}-A^{m1s}-G^{e2s}-C^{m1s}-C^{mls}-C^{e2s}-C^{mls}-A^{mls}-G^{e2s}-G^{mlt}-H (DUX4-035) (SEQ ID NO: 2)

The subject oligonucleotide was synthesized with an automated nucleic acid synthesizer (BioAutomation's MerMade 192X) by the phosphoramidite method (Nucleic Acids Research, 12, 4539 (1984)). It should be noted here that a solution obtained by adding 1-Methylimidazole (Wako Pure Chemical; product No. 134-12801) at 0.4% to Activator Solution-3 (0.25 mol/L 5-Benzylthio-1H-tetrazole- Acetonitrile Solution; Wako Pure Chemical; product No. 013-20011) was used for condensation and that about 10 minutes was set as the reaction time. As a thiolation reagent for formation of phosphorothioate bond, phenylacetyl disulfide (Carbosynth; product No. FP07495) was dissolved in a 1:1 (v/v) solution of acetonitrile (dehydrated; Kanto Chemical Co., Inc.; product No. 01837-05) and pyridine (dehydrated; Kanto Chemical Co., Inc.; product No. 11339-05) to give a concentration of 0.2 M. Other reagents used were Cap A for AKTA (1-Methylimidazole-Acetonitrile Solution; Sigma-Aldrich; product No. L040050), Cap B1 for AKTA (Acetic Anhydride-Acetonitrile Solution; Sigma-Aldrich; product No. L050050), Cap B2 for AKTA (Pyridine-Acetonitrile Solution; Sigma-Aldrich; product No. L050150), and DCA Deblock (Dichloroacetic Acid-Toluene Solution; Sigma-Aldrich; product No. L023050). As amidite reagents, 2'-O-Me nucleoside phosphoramidites (for adenosine: product No. ANP-5751; for cytidine: product No. ANP-5752; for guanosine: product No. ANP-5753; for uridine: product No. ANP-5754) were products from ChemGenes. Non-natural phosphoramidites used were the following compounds disclosed in the indicated Examples of Japanese Unexamined Patent Publication No. 2000-297097: Example 14 (5'-O-dimethoxytrityl-2'-O,4'-C-ethylene-6-N-benzoyladenosine-3'-O-(2-cyanoethyl N,N-diisopropyl)phosphoramidite); Example 27 (5'-O-dimethoxytrityl-2'-O,4'-C-ethylene-2-N-isobutylguanosine-3'-O-(2-cyanoethyl N,N-diisopropyl)phosphoramidite); Example 22 (5'-O-dimethoxytrityl-2'-O,4'-C-ethylene-4-N-benzoyl-5-methylcytidine-3'-O-(2-cyanoethyl N,N- diisopropyl)phosphoramidite); and Example 9 (5'-O-dimethoxytrityl-2'-O,4'-C-ethylene-5-methyluridine-3'-O-(2-cyanoethyl N,N-diisopropyl)phosphoramidite). Glen Unysupport^{™} FC 96 well format 0.2 µmol (GlenResearch) was used as a solid-phase carrier to synthesize the subject compound.

Protected oligonucleotide analogs with the sequence of interest were treated with 600 µl of concentrated aqueous ammonia to thereby cut out the oligomers from the support and, at the same time, remove the protective group cyanoethyl on phosphorus atoms and the protective group on nucleobases. The resultant solution containing the oligomer mixture was mixed with 300 µl of Clarity QSP DNA Loading Buffer (Phenomenex) and charged on Clarity SPE 96 well plates (Phenomenex). One milliliter of Clarity QSP DNA Loading Buffer : water = 1:1 solution, 2 mL of 0.1 M tetrabutylammonium bromide aqueous solution : acetonitrile = 8:2 (v/v) solution, 3 mL of 3% dichloroacetic acid (DCA) aqueous solution, 4 mL of water and 2 mL of 20 mM Tris aqueous solution were added in this order, and then components extracted with a 9:1 solution of 20 mM Tris aqueous solution and acetonitrile were collected. After distilling off the solvent, the compound of interest was obtained. When analyzed by reversed-phase HPLC [column (Phenomenex, Clarity 2.6 µm Oligo-MS 100A (2.1x50 mm)), Solution A: an aqueous solution of 100 mM hexafluoroisopropanol (HFIP) and 8 mM trimethylamine , Solution B: methanol, B%: from 10% to 25% (4 min, linear gradient); 60°C; 0.5 mL/min; 260 nm)], the subject compound was eluted at 3.384 min. The compound was identified by negative-ion ESI mass spectrometry (theoretical: 6446.76, found: 6446.75).

The nucleotide sequence of the subject compound is a sequence complementary to nucleotide Nos. 595-612 of Homo sapiens clone 60-1 double homeodomain protein DUX4-fl (DUX4) mRNA (NCBI-GenBank accession No. HQ266761).

It should be noted here that nucleotide sequences are shown in the SEQUENCE LISTING without distinction between natural nucleosides and 2'-O,4'-C-ethylene nucleosides.

### [Examples 2-47]

The compounds of Examples 2-47 as shown in Table 1 were synthesized in the same manner as for the compound of Example 1.

**[Table 1]**

| Example | Sequence Designation | Sequence (5'-3') | Target Region of DUX4-fl | | Molecular Weight (found) | SEQ ID NO |
|---|---|---|---|---|---|---|
| | | | 5' end position | 3' end position | | |
| 2 | DUX4-004 | aGggTgaCccCcgCcgGg | 502 | 519 | 6453.81 | 3 |
| 3 | DUX4-005 | aGcaGggTgaCccCcgCc | 505 | 522 | 6397.50 | 4 |
| 4 | DUX4-036 | gAgcAggGugAccCccGc | 506 | 523 | 6391.74 | 5 |
| 5 | DUX4-037 | gGagCagGguGacCccCg | 507 | 524 | 6459.79 | 6 |
| 6 | DUX4-006 | gGgaGcaGggTgaCccCc | 508 | 525 | 6461.68 | 7 |
| 7 | DUX4-038 | aGggAgcAggGugAccCc | 509 | 526 | 6455.77 | 8 |
| 8 | DUX4-039 | gAggGagCagGguGacCc | 510 | 527 | 6509.78 | 9 |
| 9 | DUX4-007 | cGagGgaGcaGggTgaCc | 511 | 528 | 6510.23 | 10 |
| 10 | DUX4-040 | aCgaGggAgcAggGugAc | 512 | 529 | 6519.78 | 11 |
| 11 | DUX4-041 | cAcgAggGagCagGguGa | 513 | 530 | 6519.78 | 12 |
| 12 | DUX4-008 | cCacGagGgaGcaGggTg | 514 | 531 | 6508.41 | 13 |
| 13 | DUX4-042 | cCcaCgaGggAgcAggGu | 515 | 532 | 6469.79 | 14 |
| 14 | DUX4-043 | aCccAcgAggGagCagGg | 516 | 533 | 6492.81 | 15 |
| 15 | DUX4-009 | gAccCacGagGgaGcaGg | 517 | 534 | 6478.80 | 16 |
| 16 | DUX4-044 | cGacCcaCgaGggAgcAg | 518 | 535 | 6452.79 | 17 |
| 17 | DUX4-045 | gCgaCccAcgAggGagCa | 519 | 536 | 6466.81 | 18 |
| 18 | DUX4-010 | gGcgAccCacGagGgaQc | 520 | 537 | 6454.74 | 19 |
| 19 | DUX4-046 | aGgcGacCcaCgaGggAg | 521 | 538 | 6492.80 | 20 |
| 20 | DUX4-047 | aAggCgaCccAcgAggGa | 522 | 539 | 6476.81 | 21 |
| 21 | DUX4-011 | gAagGcgAccCacGagGg | 523 | 540 | 6478.78 | 22 |
| 22 | DUX4-048 | cÇiaaGgcGac.r:::ca.r:::gaGg | 524 | 541 | 6452.79 | 23 |
| 23 | DUX4-049 | gCgaAggCgaCccAcgAg | 525 | 542 | 6466.80 | 24 |
| 24 | DUX4-012 | gGcgAagGcgAccCacGa | 526 | 543 | 6446.07 | 25 |
| 25 | DUX4-050 | gGgcGaaGgcGacCcaCg | 527 | 544 | 6468.80 | 26 |
| 26 | DUX4-051 | uGggCgaAggCgaCccAc | 528 | 545 | 6443.79 | 27 |
| 27 | DUX4-013 | gTggGcgAagGcgAccCa | 529 | 546 | 6480.71 | 28 |
| 28 | DUX4-052 | uGugGgcGaaGgcGacCc | 530 | 547 | 6432.73 | 29 |
| 29 | DUX4-053 | gTguGggCgaAggCgaCc | 531 | 548 | 6514.79 | 30 |
| 30 | DUX4-014 | gGugTggGcgAagGcgAc | 532 | 549 | 6512.74 | 31 |
| 31 | DUX4-054 | cGguSugGgcGaaGocGa | 533 | 550 | 6498.74 | 32 |
| 32 | DUX4-055 | cCggTguGggCgaAggCg | 534 | 551 | 6530.77 | 33 |
| 33 | DUX4-015 | gCcgGugTggGcgAagGc | 535 | 552 | 6512.99 | 34 |
| 34 | DUX4-056 | cGccGguGugGgcGaaGg | 536 | 553 | 6474.72 | 35 |
| 35 | DUX4-057 | gCgcCggTguGggCgaAg | 537 | 554 | 6530.79 | 36 |
| 36 | DUX4-016 | cGcgCcgGugTggGcgAa | 538 | 555 | 6473.80 | 37 |
| 37 | DUX4-058 | aCgcGccGguGugGgcGa | 539 | 556 | 6448.73 | 38 |
| 38 | DUX4-025 | gAgcCccAggCgcGcaGg | 578 | 595 | 6505.55 | 39 |
| 39 | DUX4-026 | cAggCgcGcaGggCacGu | 581 | 598 | 6452.80 | 40 |
| 40 | DUX4-031 | cCcaGgcGcgCagGgcAc | 583 | 600 | 6404.77 | 41 |
| 41 | DUX4-027 | cCccAggCgcGcaGggCa | 584 | 601 | 6423.84 | 42 |
| 42 | DUX4-032 | aGccCcaGgcGcgCagGg | 586 | 603 | 6444 .78 | 43 |
| 43 | DUX4-028 | gAgcCccAggCgcGcaGg | 587 | 604 | 6452.28 | 44 |
| 44 | DUX4-033 | gAgaGccCcaGgcGcgCa | 589 | 605 | 6428.80 | 45 |
| 45 | DUX4-029 | gGagAgcCccAggCgcGc | 590 | 607 | 6449.79 | 46 |
| 46 | DUX4-034 | uGggAgaGccCcaGgcGc | 592 | 609 | 6431.75 | 47 |
| 47 | DUX4-030 | gTggGagAgcCccAggCg | 593 | 610 | 6504.46 | 48 |

In the sequences shown in the Table, small letters indicate 2'-OMe-RNA, and underlined capital letters indicate ENA. It should be noted here that the nucleobase of C in ENA is 5-methylcytosine. All the bonds between nucleosides are phosphorothioate bonds. The target region is defined with nucleotide numbers of Homo sapiens clone 60-1 double homeodomain protein DUX4-fl (DUX4) mRNA (NCBI-GenBank accession No.HQ266761). The molecular weight indicates values actually found by negative-ion ESI mass spectrometry.

### [Examples 48-95]

The compounds as shown in Table 2 below can also be synthesized under the same conditions as used in Example 1.

**[Table 2]**

| Example | Sequence Designation | Sequence (5'-3') | Target Region of DUX4-fl | | SEQ ID NO |
|---|---|---|---|---|---|
| | | | 5' end position | 3'end position | |
| 48 | DUX4-060 | gAgcAgggTgaCccCcgC | 506 | 523 | 5 |
| 49 | DUX4-0 61 | ggAgCagggTgAcCccCg | 507 | 524 | 6 |
| 50 | DUX4-062 | gggAgCagggTgAccCcC | 508 | 525 | 7 |
| 51 | DUX4-0 63 | aCgAgggAgcAgggTgaC | 512 | 529 | 11 |
| 52 | DUX4-0 64 | gAccCaCgagggAgCAgg | 517 | 534 | 16 |
| 53 | DUX4-0 65 | CgaCccAcgAgggAgcAg | 518 | 535 | 17 |
| 54 | DUX4-066 | gAaggCgAccCaCgAggg | 523 | 540 | 22 |
| 55 | DUX4-067 | CgaAggCgacCcaCgAgg | 524 | 541 | 23 |
| 56 | DUX4-068 | TgTgggCgaAggcgAcCc | 530 | 547 | 29 |
| 57 | DUX4-069 | gTgTgggCgaAggCgaCc | 531 | 548 | 30 |
| 58 | DUX4-070 | ggTgTgggCgAaggCgaC | 532 | 549 | 31 |
| 59 | DUX4-071 | AgcAgggTgaCccCcgC | 506 | 522 | 49 |
| 60 | DUX4-072 | gAgcAgggTgaCcCcCg | 507 | 523 | 50 |
| 61 | DUX4-073 | ggAgcAgggTgaCcCcC | 508 | 524 | 51 |
| 62 | DUX4-074 | gggAgcAgggTgACcC | 509 | 525 | 52 |
| 63 | DUX4-075 | CgAgggAgcAgggTgaC | 512 | 528 | 53 |
| 64 | DUX4-076 | aCgAgggAgcAgggTgA | 513 | 529 | 54 |
| 65 | DUX4-077 | AcCcaCgAgggAgcAgg | 517 | 533 | 55 |
| 66 | DUX4-078 | gAccCaCgagggAgCAg | 518 | 534 | 56 |
| 67 | DUX4-079 | CgaCccAcgAgggAgcA | 519 | 535 | 57 |
| 68 | DUX4-080 | AaggCgaCcCaCgAggg | 523 | 539 | 58 |
| 69 | D0X4-081 | gAaggCgaCcCaCgAgg | 524 | 540 | 59 |
| 70 | DUX4-082 | CgaAggCgaCccAcgAg | 525 | 541 | 60 |
| 71 | BUX4-083 | gTgggCgaAggCgaCcC | 530 | 546 | 61 |
| 72 | DUX4-084 | TgTgggCgaAggCgaCc | 531 | 547 | 62 |
| 73 | DUX4-085 | gTgTgggCgaAggCgaC | 532 | 548 | 63 |
| 74 | DUX4-086 | ggTgTgggCgaAggCgA | 533 | 549 | 64 |
| 75 | DUX4-087 | gCagggTgaCccCcgC | 506 | 521 | 65 |
| 76 | DUX4-088 | AgcAgggTgacCccCg | 507 | 522 | 66 |
| 77 | DUX4-089 | gAgcAgggTgacCccC | 508 | 523 | 67 |
| 78 | DUX4-090 | ggAgcAgggTgaCccC | 509 | 524 | 68 |
| 79 | DUX4-091 | gggAgcAgggTgaCcC | 510 | 525 | 69 |
| 80 | DUX4-092 | gAgggAgcAgggTgaC | 512 | 527 | 70 |
| 81 | DUX4-093 | CgAgggAgcAgggTga | 513 | 528 | 71 |
| 82 | DUX4-094 | AcgAgggAgCagggTg | 514 | 529 | 72 |
| 83 | DUX4-095 | CccAcgAgggAgcAgg | 517 | 532 | 73 |
| 84 | DUX4-096 | AccCaCgagggAgcAg | 518 | 533 | 74 |
| 85 | DUX4-097 | gAccCacgAgggAgcA | 519 | 534 | 75 |
| 86 | DUX4-0 98 | CgaCccAcgAgggAgc | 520 | 535 | 76 |
| 87 | DUX4-099 | AggCgaCccAcgAggg | 523 | 538 | 77 |
| 88 | DUX4-100 | AaggCgaCccAcgAgg | 524 | 539 | 78 |
| 89 | DUX4-101 | gAaggCgaCccAcgAg | 525 | 540 | 79 |
| 90 | DUX4-102 | CgaAggcgaCccAcgA | 526 | 541 | 80 |
| 91 | DUX4-103 | TgggCgaAggCgacCc | 530 | 545 | 81 |
| 92 | DUX4-104 | gTgggCgaAggcgAcC | 531 | 546 | 82 |
| 93 | D0X4-105 | TgTgggcgAaggCgAc | 532 | 547 | 83 |
| 94 | DUX4-106 | gTgTgggcgAaggCgA | 533 | 548 | 84 |
| 95 | DUX4-107 | ggTgTgggCgaAggCg | 534 | 549 | 85 |

In the sequences shown in the Table, small letters indicate 2'-OMe-RNA, and underlined capital letters indicate ENA. It should be noted here that the nucleobase of C in ENA is 5-methylcytosine. All the bonds between nucleosides are phosphorothioate bonds. The target region is defined with nucleotide numbers of Homo sapiens clone 60-1 double homeodomain protein DUX4-fl (DUX4) mRNA (NCBI-GenBank accession No.HQ266761). The molecular weight indicates values actually found by negative-ion ESI mass spectrometry.

As used herein, A^{t}, G^{t}, 5meC^{t}, C^{t}, T^{t}, U^{t}, A^{p}, G^{p}, 5meC^{p}, C^{p}, T^{p}, U^{p}, A^{s}, G^{s}, 5meC^{s}, C^{s}, T^{s}, U^{s}, A^{mlt}, G^{mlt}, C^{mlt} 5meC^{mlt}, U^{mlt}, A^{mlp}, G^{mlp}, C^{mlp}, 5meC^{mlp}, U^{mlp}, A^{mls}, G^{mls}, C^{mls}, 5meC^{mls}, U^{mls}, A^{2t}, G^{2t}, C^{2t}, T^{2t}, A^{e2p}, G^{e2p}, C^{e2p}, T^{e2p}, A^{e2s}, G^{e2s}, C^{e2s}, T^{e2s}, A^{lt}, G^{lt}, C^{lt}, T^{lt}, A^{elp}, G^{elp}, C^{elp}, T^{elp}, A^{els}, G^{els}, C^{els}, T^{els}, A^{3t}, G^{3t}, C^{3t}, T^{3t}, A^{e3p}, G^{e3p}, C^{e3p}, T^{e3p}, A^{e3s}, G^{e3s}, C^{e3s}, T^{e3s}, A^{m2t}, G^{m2t}, 5meC^{m2t}, T^{m2t}, A^{m2p}, G^{m2p}, 5meC^{m2p}, T^{m2p}, A^{m2s}, G^{m2s}, 5meC^{m2s}, and T^{m2s} represent groups whose respective structures are shown below.

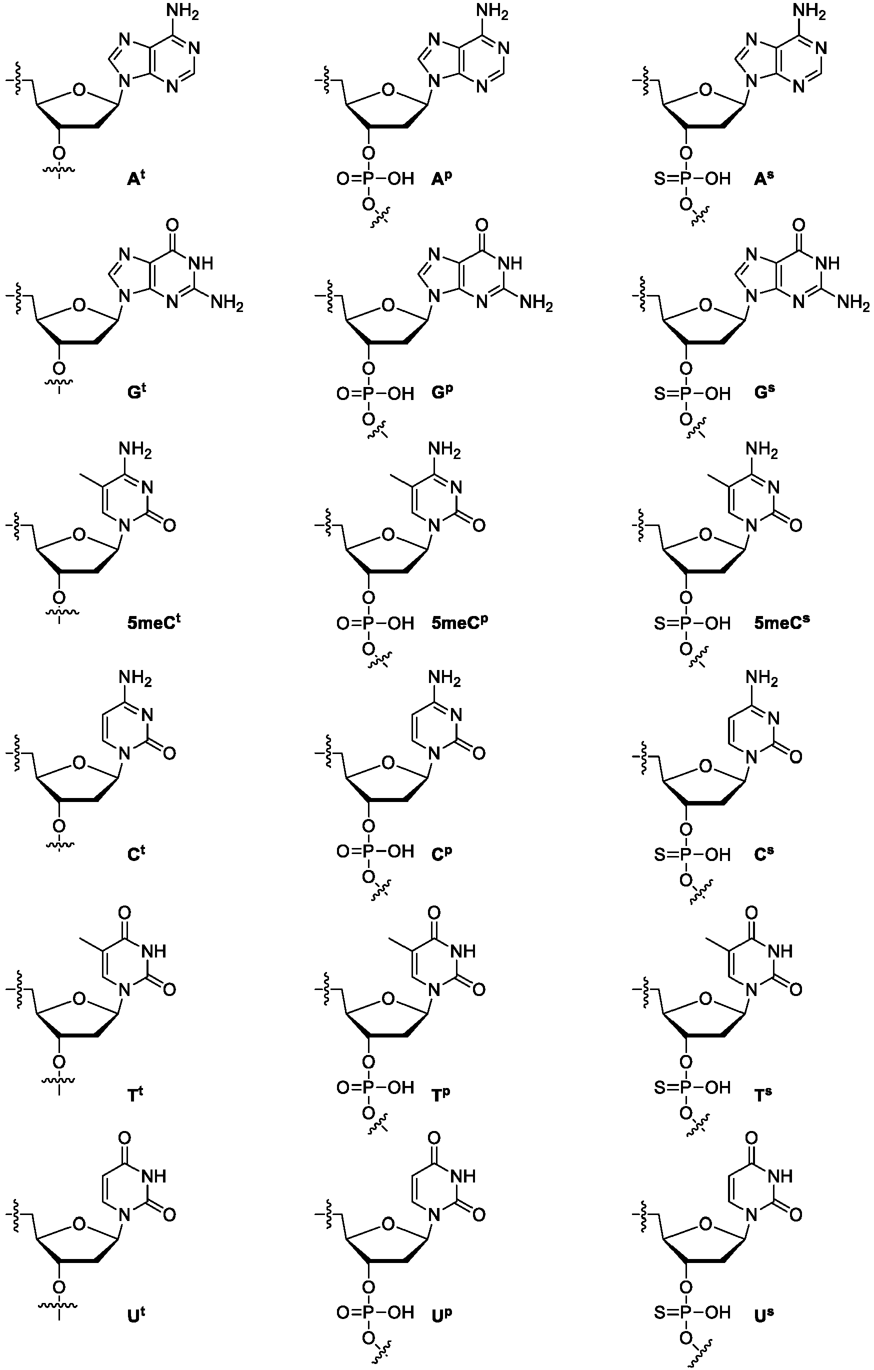

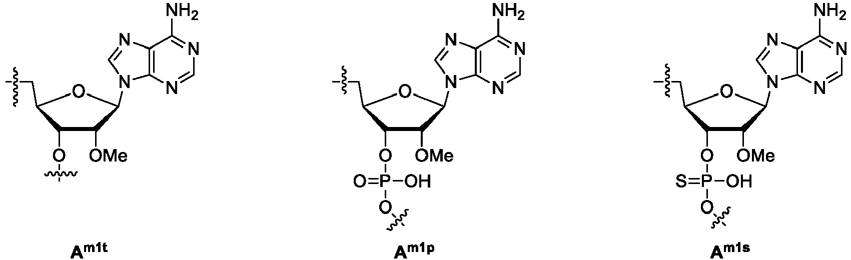

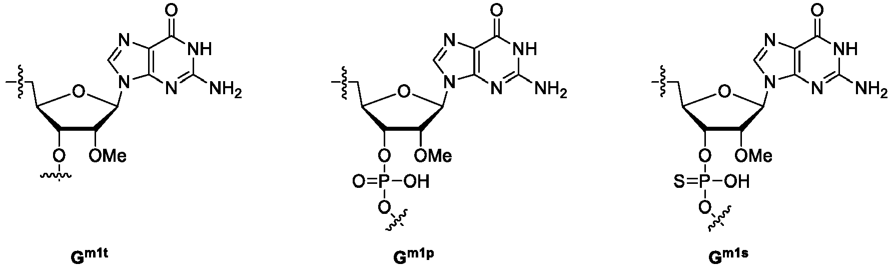

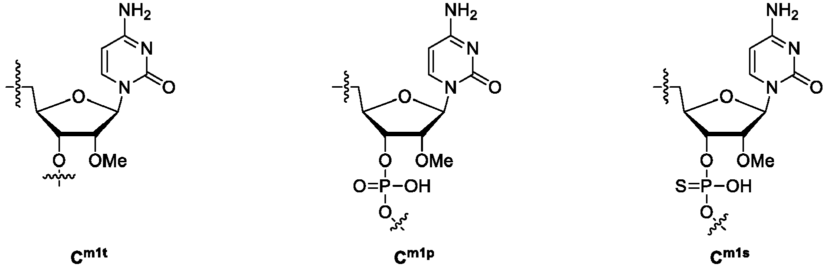

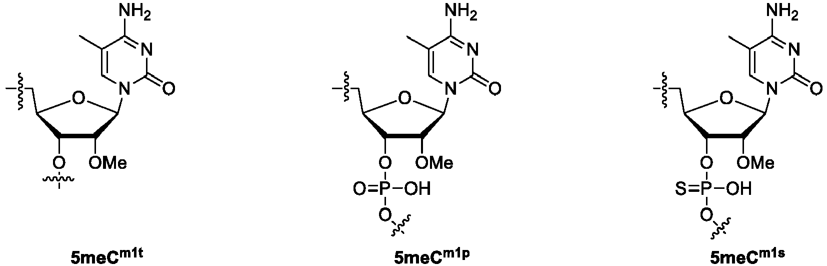

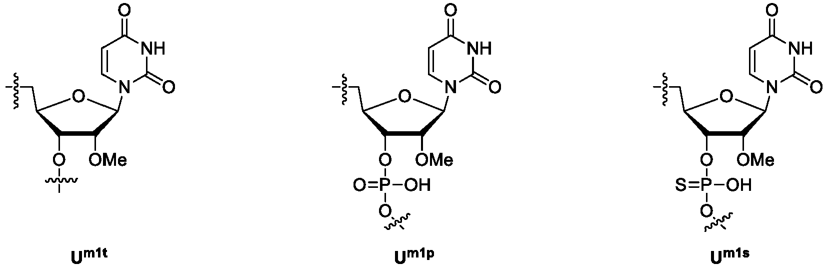

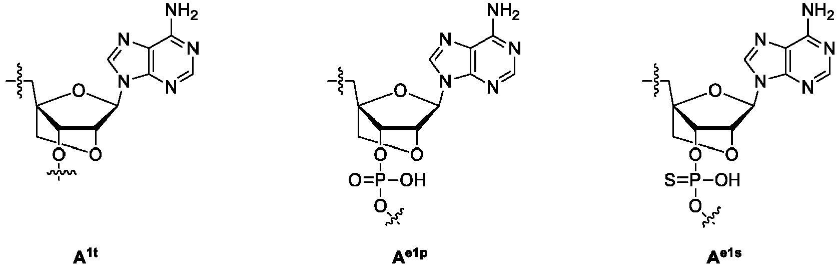

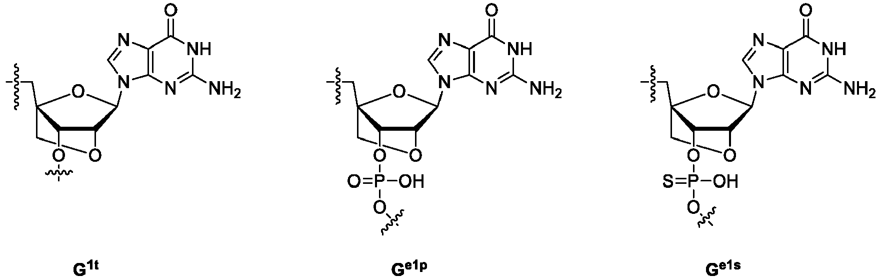

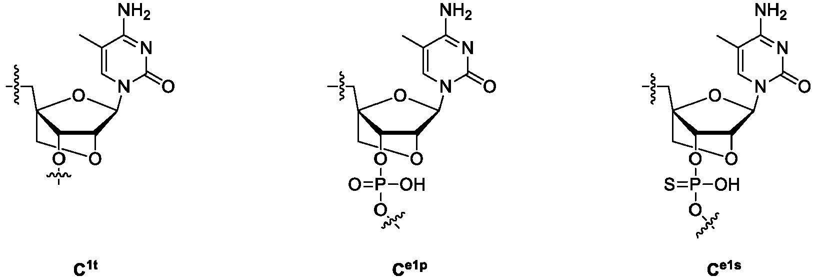

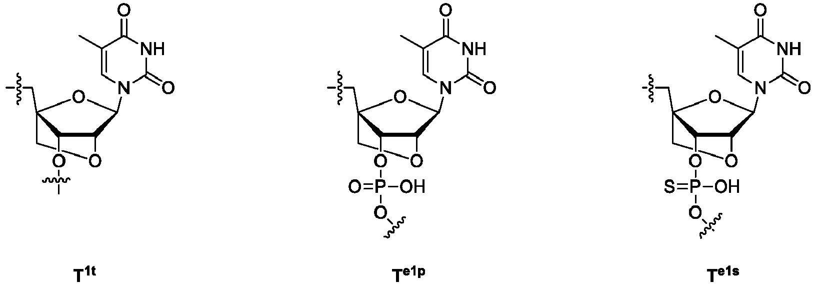

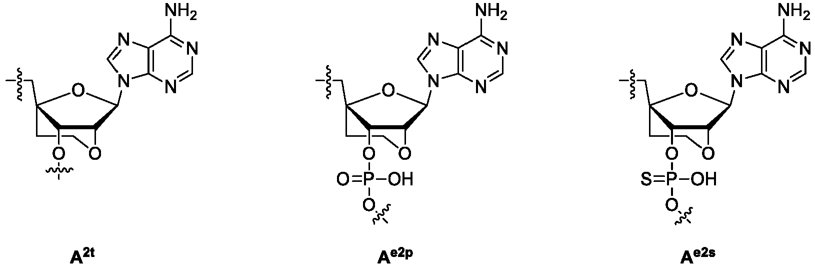

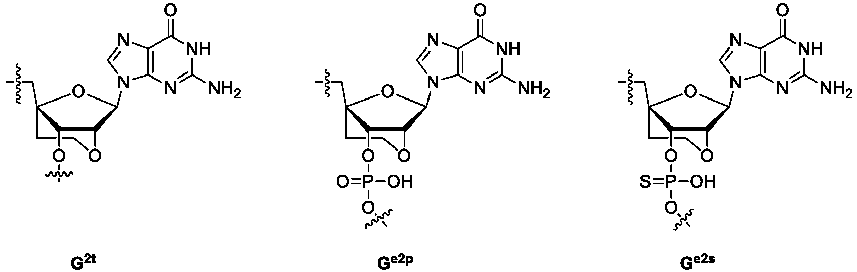

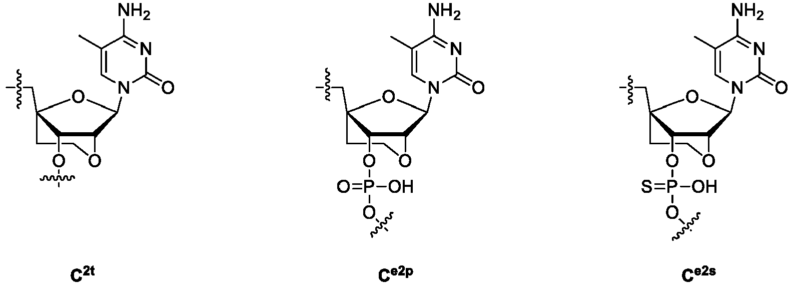

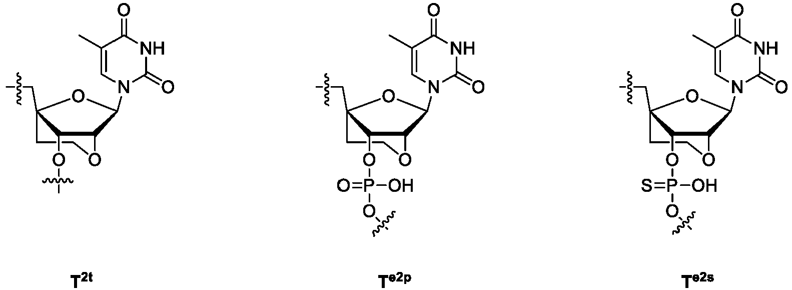

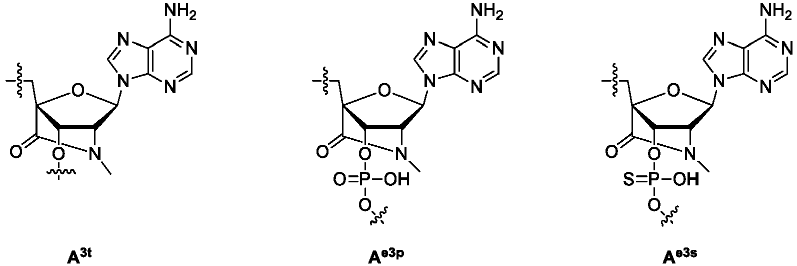

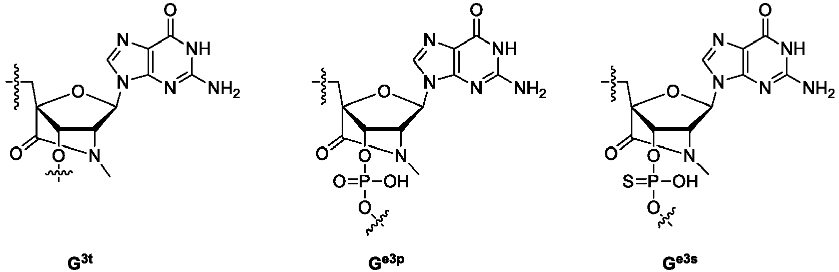

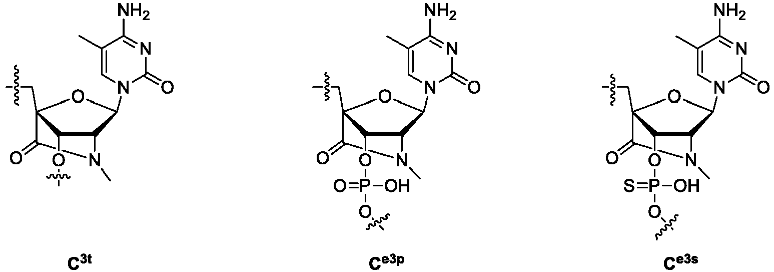

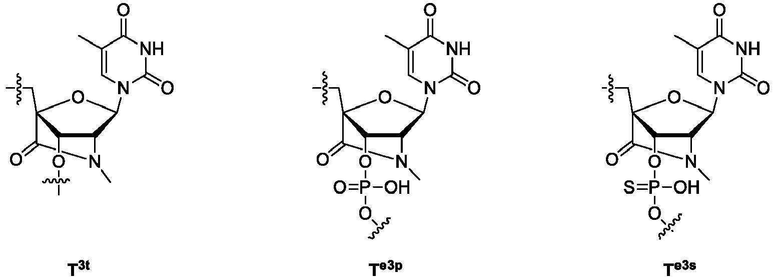

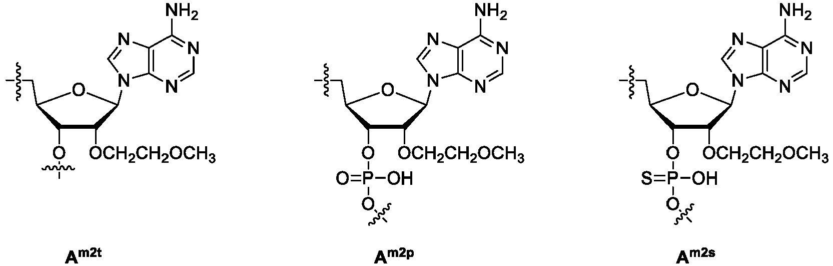

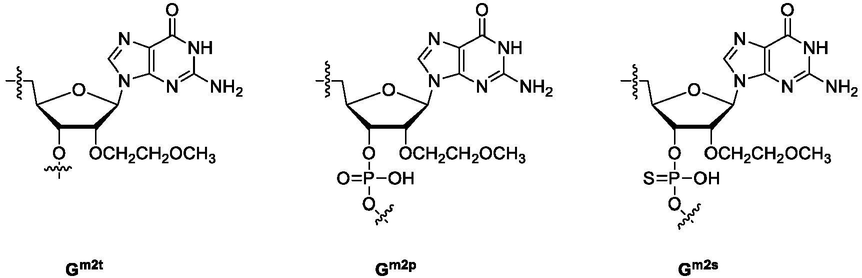

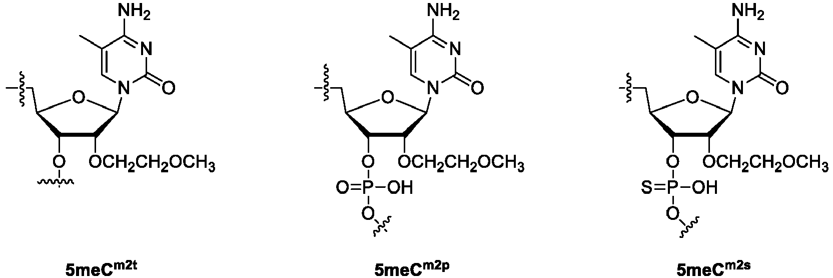

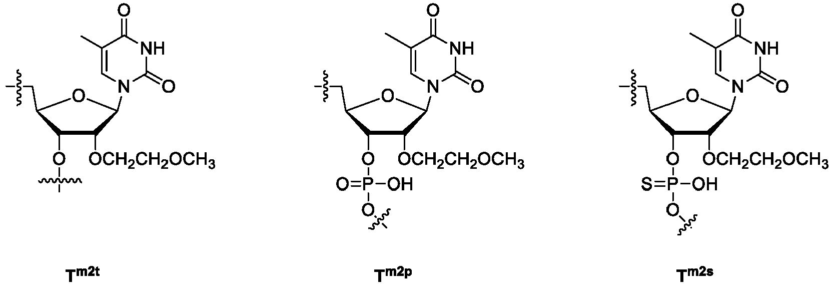

### [Test Example 1]

### (A) Construction of DUX4 Minigene Construct

A recombinant E. *coli* strain harboring BAC clone RP11-242C23 containing the DUX4 gene (Morioka et al., PLoS One 2016, 11: e0151963) was purchased from the BAC PAC Resources Center, Children's Hospital Oakland. Cells were cultured in LB/chloramphenicol agar medium, and then BAC DNA was purified using QIAGEN plasmid midi kit (QIAGEN, cat.no.12143) according to the protocol QP01.

A region containing exon 1 to exon 3 (2163 bps) of the DUX4 gene was amplified by PCR using the BAC DNA as a template and primer 207 (5'-CGCGTCCGTCCGTGAAATTCC-3') (SEQ ID NO: 86), primer 209 (5'-CAGGGGATATTGTGACATATCTCTGCAC-3') (SEQ ID NO: 87), and PrimeStar GXL (Takara; cat.no.R050A). The PCR product was purified using MinElute Gel Extraction Kit (QIAGEN, cat.no.28606) and ligated to pCR blunt vector (Thermo Fisher Scientific; cat.no.K275020) using Mighty mix (Takara; cat.no.6023). *E. coli* TOP10 competent cells (Thermo Fisher; cat.no.C404010) were transformed by the resultant recombinant plasmid and subjected to the selection on a LB/kanamycin agar plate. Transformant colonies thus selected were cultured in a liquid medium, and the recombinant plasmid was purified from the culture using GenElute plasmid miniprep kit (SIGMA; cat.no.PLN350). The nucleotide sequence of the recombinant plasmid obtained was determined with ABI 3500xL Genetic Analyzer (Applied Biosystems) to confirm the insertion of the region containing exon 1 to exon 3 of the DUX4 gene into the recombinant plasmid. This plasmid clone was designated as plasmid 318.

PCR with PrimeStar GXL was conducted again using plasmid 318 as a template, primer 216 containing EcoRI recognition sequence and kozak sequence (5'-GAATTCTGCCACCATGGCCCTCCCG-3') (SEQ ID NO: 88) and primer 218 containing XhoI recognition sequence (5'-CTCGAGCTATAGGATCCACAGGGAGG-3') (SEQ ID NO: 89) to thereby add EcoRI recognition sequence and kozak sequence to the 5' end and XhoI recognition sequence to the 3'end of the DNA fragment containing exons 1 to 3 of the DUX4 gene. The resultant DNA fragment with restriction enzyme recognition sequences and kozak sequence was ligated to pCR blunt vector to generate a plasmid clone designeted as plasmid 320.

Plasmid 320 and expression vector pcDNA3.1(+) (Thermo Fisher Scientific; cat.no.V79020) were treated with EcoRI and XhoI at 37 °C for 2 hrs, respectively, and subjected to agarose gel electrophoresis. The bands corresponding to the DNA fragment containing the DUX4 gene and pcDNA3.1(+) vector were cut out from the gel and treated with MinElute Gel Extraction Kit (QIAGEN, cat.no.28606) to purify the DNA fragment and the vector. Subsequently, the DNA fragment containing the DUX4 gene and the vector were ligated using Mighty mix. The ligated product was designated as the DUX4 minigene construct after the confirmation of its nucleotide sequence with ABI 3500xL Genetic Analyzer.

### (B) Transfection of DUX4 Minigene Construct and the Compounds of Examples into HeLa Cells

HeLa cells, which is a human cervical cancer-derived cultured cell line, were purchased from RIKEN BRC Cell Bank (cat. RCB0007, Tsukuba, Japan). HeLa cells were cultured in the DMEM (SIGMA; cat.no.D5796) containing 10% FBS (Thermo Fisher Scientific; cat.no.10270-106) at 37°C under CO₂ concentration of 5%. HeLa cells were plated on 6-well dishes (Thermo Fisher Scientific; cat.no.140675) at 56 x 10⁴ cells. The next day, 1 µg of DUX4 minigene construct and the compound of Example (final concentration: 100 nM) were transfected into HeLa cells using Lipofectamine 2000 (Thermo Fisher Scientific; cat.no.11668027).

### (C) Detection of Transcription Products from the DUX4 Minigene Construct

Twenty-four hours after transfection of the DUX4 minigene construct, cells were collected and RNA was extracted therefrom with RNeasy mini kit (QIAGEN; cat.no.74104). Reverse transcription was performed with PrimeScript (Takara; cat.no.2680A). Subsequently, PCR was performed using primer 222: 5'-GGATTCAGATCTGGTTTCAGAATCGAAGG-3' (SEQ ID NO: 90) and primer 225: 5'-CCAGGAGATGTAACTCTAATCCAGGTTTGC-3' (SEQ ID NO: 91) to amplify both DUX4-fl and DUX4-s cDNA. The RT-PCR products were separated by 4.8% polyacrylamide gel electrophoresis, and visualized with LAS3000 (Fuji Film). As molecular size markers, λ/EcοT14I marker (Takara; cat.no.3010 was treated with Takara; cat.no. 1038A for use; indicated as M1 in Figs.) and 100 bp marker (Takara; cat.no.3422B; indicated as M2 in Figs.) were used.

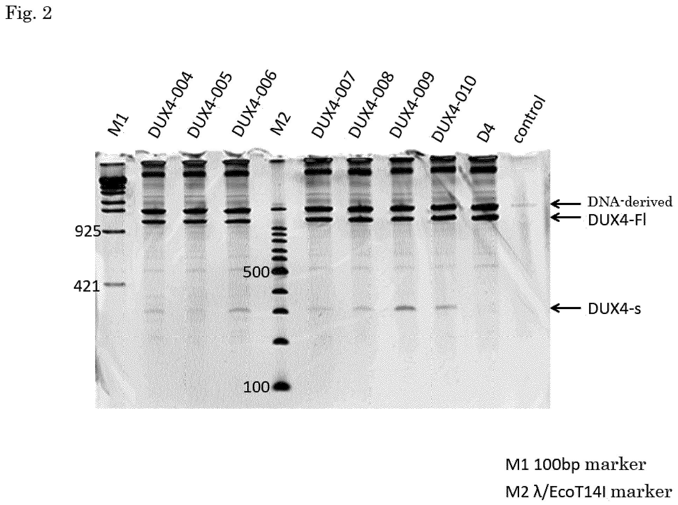

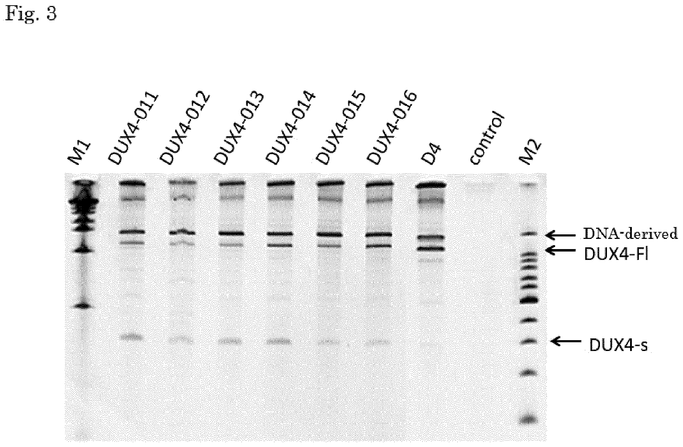

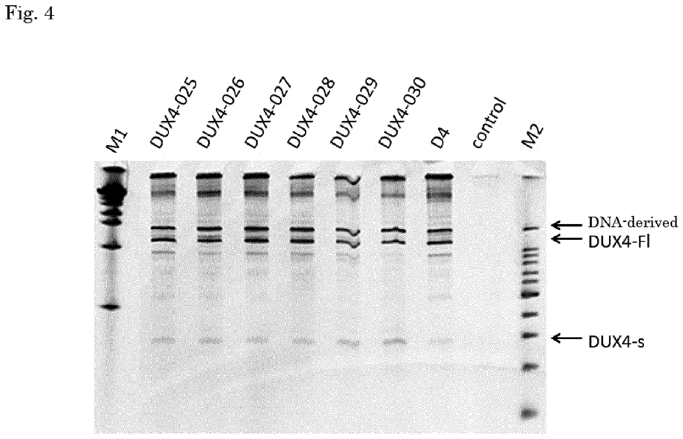

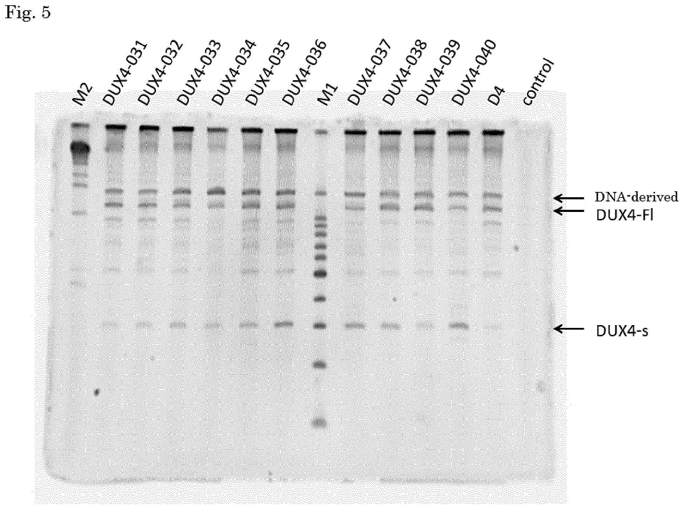

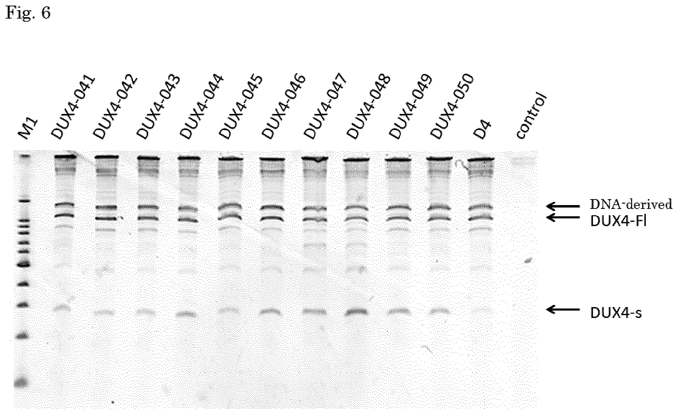

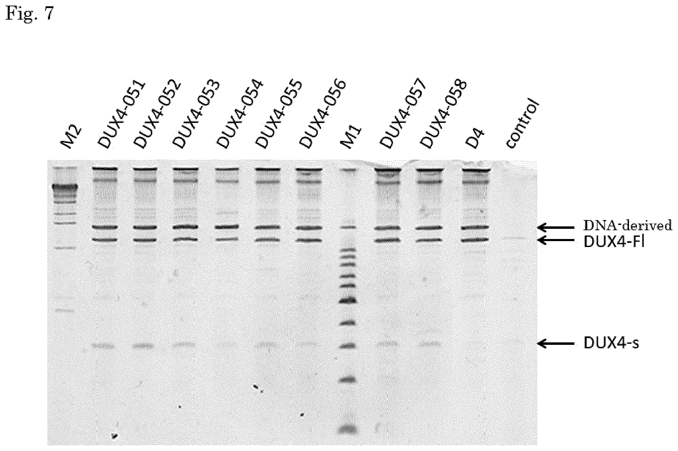

The results of gel electrophoresis are shown in Figs. 2 to 7. In these Figs., "control" represents a sample prepared from cells not transfected with either the DUX4 minigene construct or the compound of Example. "D4" represents a sample from cells transfected with the DUX4 minigene construct alone. In the samples prepared from cells transfected with both the DUX4 minigene construct and the compounds of Examples (DUX4-004 to DUX4-016, and DUX4-025 to DUX4-058), bands of approx. 300 bp which seem to be derived from DUX4-s were observed and the intensities of these bands were stronger than that observed in the D4 lane.

### (D) Confirmation of the Nucleotide Sequence of the DUX4-s Band Generated in the presence of the Compound of Example

Twenty-four hours after transfection of the DUX4 minigene construct, cells were collected and RNA was extracted therefrom with RNeasy mini kit. Reverse transcription was performed with PrimeScript and then PCR was performed using primers 222 and 225 which amplify both DUX4-fl and DUX4-s. Bands of the resultant PCR products derived from the DUX4-fl and DUX4-s were separated by 2% agarose gel electrophoresis. A band of approx. 300 bp was cut out and purified with MinElute Gel Extraction kit. The purified DNA was ligated to pCR blunt vector using Mighty mix. *E. coli* TOP10 competent cells were transformed by the recombinant plasmid and subjected to the selection on a LB/kanamycin agar plate. Transformant colonies were cultured in a liquid medium, and the recombinant plasmid was purified using GenElute plasmid miniprep kit. Using the recombinant plasmid thus obtained as a template, the nucleotide sequence of the 300bp fragment was deterimened by the Sanger method using M13 forward primer (5'-CGACGTTGTAAAACGACGGCCAGT-3') (SEQ ID NO: 92) and M13 reverse primer (5'-ggaaacagctatgaccatgattac-3') (SEQ ID NO: 93) and with ABI 3500xL Genetic Analyzer. The results revealed that 10 bp at the 3' end of exon 2 were deleted in the obtained sequence, compared to the sequence of DUX4-s reported (GenBank HQ266762). Since the stop codon of DUX4-s is located at the beginning of exon 2, it is considered that full-length DUX4-s protein (GenBank: ADN68617.1) is also produced from the sequence obtained in this experiment.

### (E) Measurement of the Expression Levels of the DUX4's Target Genes

Twenty-four hours after transfection of the DUX4 minigene construct and the compound of Example (DUX4-009), cells were collected and RNA was extracted with RNeasy mini kit. Reverse transcription was performed with PrimeScript to synthesize cDNA. Using the resultant cDNA as a template, real time PCR was performed with PowerUP SYBR Green PCR master mix (Thermo Fisher Scientific; cat.no. A25742) to measure the expression levels of ZSCAN4, MBD3L2 and TRIM43 which are known as DUX4's target genes for transcriptional activation. For the PCR, StepOne Plus (Thermo Fisher Scientific) was used. Data were converted to numerical form by the ΔΔCt method using the RPL13A gene as an internal standard. The sequences of the primers used are as shown below.
ZSCAN4-Fw: 5'-TGGAAATCAAGTGGCAAAAA-3' (SEQ ID NO: 94); ZSCAN4-Rv: 5'-CTGCATGTGGACGTGGAC-3' (SEQ ID NO: 95)
MBD3L2-Fw: 5'-GCGTTCACCTCTTTTCCAAG-3' (SEQ ID NO: 96); MBD3L2-Rv: 5'-GCCATGTGGATTTCTCGTTT-3' (SEQ ID NO: 97)
TRIM43-Fw: 5'-ACCCATCACTGGACTGGTGT-3' (SEQ ID NO: 98); TRIM43-Rv: 5'-CACATCCTCAAAGAGCCTGA-3' (SEQ ID NO: 99)

The results are shown in Figs. 8A and 8B. The cells transfected with the DUX4 minigene construct and the compound of Example (Fig. 8A: DUX4-009, DUX4-031, DUX4-036 or DUX4-048; Fig. 8B: DUX4-48.7, DUX4-48.11, DUX4-48.12, DUX4-48.13 or DUX4-52.2) showed lower expression levels of ZSCAN4, MBD3L2 and TRIM43, compared to those cells transfected with DUX4 minigene construct alone.

### [Test Example 2]

### (A) Preparation of the DUX4 pre-mRNA (D4Z4 mRNA)

The DUX4 minigene construct used in above experiments with HeLa cells was digested with EcoRI and XhoI, and the resultant fragments were separated by agarose gel electrophoresis. The DNA fragment of interest was purified from the gel and ligated to pCS2-V5 vector having SP6 promoter. The plasmid DUX4_pCS2 thus obtained was linearized with NotI. Using the linearized DNA as a template, mRNA was synthesized *in vitro* with mMessage mMachine SP6 transcription kit (ThermoFisher Scientific; cat.no.AM1340) and purified with RNeasy MinElute Cleanup kit (QIAGEN; cat.no.74204).

### (B) Injection of the D4Z4 mRNA and the Compound of Example into Zebrafish, and Detection of DUX4 pre-mRNA Splice-Switching

The DUX4 pre-mRNA (final concentration: 10 ng/µl) alone or a mixture of the DUX4 pre-mRNA and the compound of Example (DUX-048, final concentration: 500 µM) was injected into about 100 fertilized eggs of wild-type zebrafish strain RIKEN WT with a microinjector (Eppendorf) in an amount of 1 nL. After 5 hrs, TRIzol reagent (ThermoFisher Scientific; cat.no. 15596026) was added to homogenize the fertilized eggs, and RNA was purified with RNeasy Mini kit. Then, DNase treatment was conducted with RNase-free DNase set (QIAGEN; cat.no.79254). Aliquots of the resultant RNA were used as RT(-) samples (negative controls). On the other hand, a 1.2 µg aliquot of the RNA was reverse transcribed using PrimeScript 1st strand cDNA synthesis kit. Using the cDNA thus obtained as a template, PCR was performed with primer 222 (SEQ ID NO: 90) and primer 225 (SEQ ID NO: 91), and with PrimeSTAR GXL DNA polymerase (Takara; cat.no.R050A). Bands of DUX4-fl and DUX4-s were separated by 4.8% polyacrylamide gel electrophoresis, and visualized with LAS3000 (Fuji Film). As molecular size markers, λ/EcοT14I marker (Takara; cat.no.3010 was treated with Takara; cat.no. 1038A for use; indicated as M1 in Figs.) and 100 bp marker (Takara; cat.no.3422B; indicated as M2 in Figs.) were used.

The results are shown in Fig. 9. As expected, no band was detected in the negative controls without reverse transcription (RT-), confirming the specificity of the primers used in this experiment. On the other hand, of the samples with reverse transcription (RT+), a band corresponding to DUX4-fl was decreased and another band corresponding to DUX4-s was increased in the sample obtained from the fertilized eggs injected with the compound of Example (DUX-048).

### [Examples 96-130]

Compounds as shown in Table 3 below were synthesized under the same conditions as used in Example 1.

**[Table 3]**

| Example | Sequence Designation | Sequence (5'-3') | Target Region of DUX4-fl | | Molecular Weight (found) | SEQ ID NO |
|---|---|---|---|---|---|---|
| | | | 3' end position | 5' end position | | |
| 96 | DUX4-48.1 | CgaAggCgaCcCaCgAgg | 524 | 541 | 6506.84 | 23 |
| 97 | DUX4-48.2 | CgAaggCgAcCcaCgAgg | 524 | 541 | 6492.86 | 23 |
| 98 | DUX4-48.3 | CgaAggCgaCccAcgAgg | 524 | 541 | 6466.81 | 23 |
| 99 | DUX4-48.4 | CgAaggCgAccCaCgagg | 524 | 541 | 6480.83 | 23 |
| 100 | DUX4-48.5 | CgAaggCgaCcCaCgAgg | 524 | 541 | 6506.88 | 23 |
| 101 | DUX4-48.6 | cgaAggCgaCccAcgAgg | 524 | 541 | 6440.77 | 23 |
| 102 | DUX4-48.7 | CgaAggCgacccAcgAgg | 524 | 541 | 6440.80 | 23 |
| 103 | DUX4-48.8 | CgAaggcgaccCaCgAgg | 524 | 541 | 6454.85 | 23 |
| 104 | DUX4-48.9 | CgaAggcgacccAcgAgg | 524 | 541 | 6414.78 | 23 |
| 105 | DUX4-48.10 | cgaAggcgaccCaCgAgg | 524 | 541 | 6428.79 | 23 |
| 106 | DUX4-48.11 | cgAaggcgAcCcacgAgg | 524 | 541 | 6414.79 | 23 |
| 107 | DUX4-48.12 | CgAaggcgacccaCgAgg | 524 | 541 | 6428.79 | 23 |
| 108 | DUX4-48.13 | cgAaggCgacCcacgAgg | 524 | 541 | 6428.80 | 23 |
| 109 | DUX4-48.14 | cgaAggcgaCccaCgAgg | 524 | 541 | 6428.82 | 23 |
| 110 | DUX4-48.15 | cgaAggcgAcccaCgAgg | 524 | 541 | 6114.81 | 23 |
| 111 | DUX4-48.16 | cgAaggcgAccCacgAgg | 524 | 541 | 6414.80 | 23 |
| 112 | DUX4-48.17 | cgAaggcgacCcAcgAgg | 524 | 541 | 6414.79 | 23 |
| 113 | DUX4-48.18 | cgAaggCgacccAcgAgg | 524 | 541 | 6414.79 | 23 |
| 114 | DUX4-48.19 | cgAaggCgaCccacgAgg | 524 | 541 | 6428.80 | 23 |
| 115 | DUX4-48.20 | cgaAggcgacccaCgAgg | 524 | 541 | 6402.77 | 23 |
| 116 | DUX4-48.21 | cgAaggcgaCccacgAgg | 524 | 541 | 6402.79 | 23 |
| 117 | DUX4-48.22 | CgaaggcgacccaCgAgg | 524 | 541 | 6416.81 | 23 |
| 118 | DUX4-48.23 | cgAaggCgacccacgAgg | 524 | 541 | 6402.77 | 23 |
| 119 | DUX4-52.1 | TgTgggCgaaggCgaCcC | 530 | 547 | 6502.80 | 100 |
| 120 | DUX4-52.2 | TgTgggCgaAggCgaCcc | 530 | 547 | 6488.81 | 100 |
| 121 | DUX4-52.3 | TgTgggCgaaggcgaCcC | 530 | 547 | 6476.81 | 100 |
| 122 | DUX4-52.4 | TgTgggcgaaggCgaCcC | 530 | 547 | 6476.81 | 100 |
| 123 | DUX4-52.5 | TgTgggcgaaggcgaCcC | 530 | 547 | 6450.78 | 100 |
| 124 | DUX4-52.6 | TgTgggcgaaggCgaccC | 530 | 547 | 6450.78 | 100 |
| 125 | DUX4-52.7 | TgugggcgaaggCgaCcC | 530 | 547 | 6450.78 | 101 |
| 126 | DUX4-52.8 | TgugggCgaAggcgaCcc | 530 | 547 | 6436.77 | 101 |
| 127 | DUX4-52.9 | ugTgggcgaaggCgAcCc | 530 | 547 | 6436.77 | 100 |
| 128 | DUX4-52.10 | TgugggcgaaggcgaCcC | 530 | 547 | 6424.78 | 101 |
| 129 | DUX4-52.11 | TgTgggcgaaggcgaCcc | 530 | 547 | 6424.78 | 100 |
| 130 | DUX4-52.12 | TgugggcgAaggcgaCcc | 530 | 547 | 6410.76 | 101 |

In the sequences shown in the Table, small letters indicate 2'-OMe-RNA, and capital letters indicate ENA. It should be noted here that the nucleobase of C in ENA is 5-methylcytosine. All the bonds between nucleosides are phosphorothioate bonds. The target region is defined with nucleotide numbers of Homo sapiens clone 60-1 double homeodomain protein DUX4-fl (DUX4) mRNA (NCBI-GenBank accession No.HQ266761). The molecular weight indicates values actually found by negative-ion ESI mass spectrometry.

### [Test Example 3]

### (A) Transfection of the DUX4 Minigene Construct and the Compounds of Examples into HeLa Cells, and Detection of Transcription Products from the DUX4 Minigene Construct

Transfection of the DUX4 minigene construct and the compounds of Examples (DUX4-048; DUX4-48.1 to DUX4-48.23; DUX4-052; and DUX4-52.1to DUX4-52.12) into HeLa cells, and detection of transcription products from the DUX4 minigene construct were performed in the same manner as described in (B) and (C) in Test Example 1.

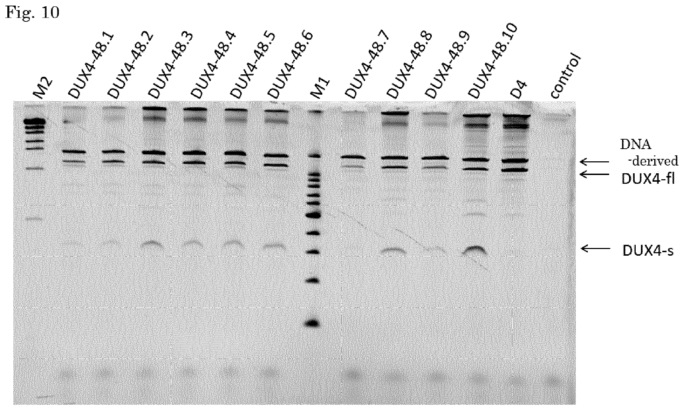

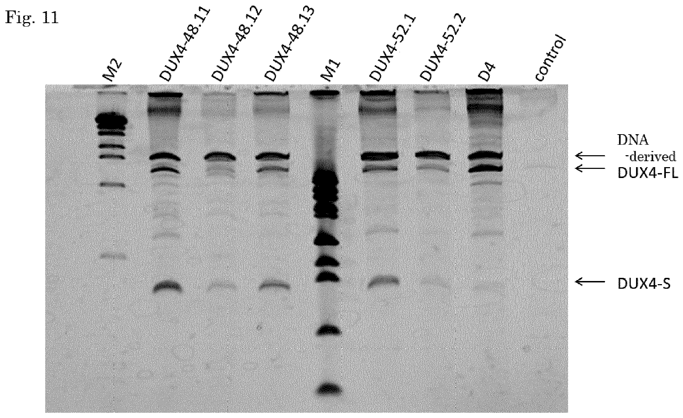

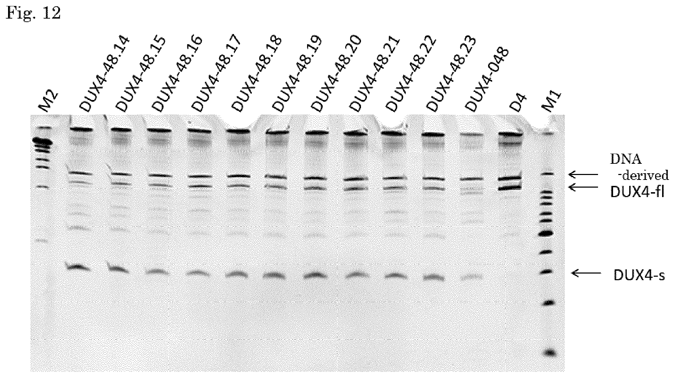

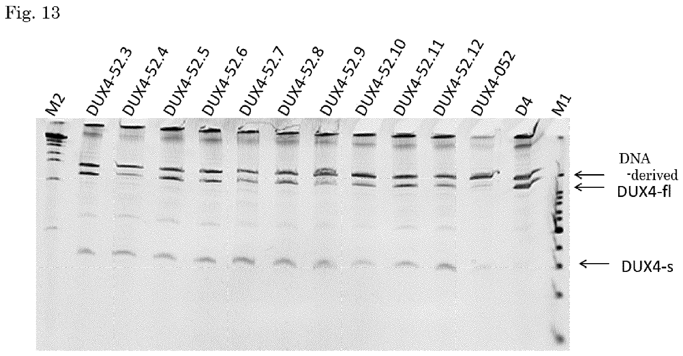

The results of gel electrophoresis are shown in Figs. 10 to 13. In these Figs., "control" represents the sample from those cells which were not transfected with either the DUX4 minigene construct or the compound of Example. "D4" represents the sample from those cells which were transfected with the DUX4 minigene construct alone. In the samples from those cells which were also transfected with the compounds of Examples (DUX4-048; DUX4-48.1 to DUX4-48.23; DUX4-052; and DUX4-52.1 to DUX4-52.12), a stronger band of approx. 300 bp derived from DUX4-s was observed than that in the D4 lane. On the other hand, in the samples from those cells which were transfected with the compounds of Examples (DUX4-48.7, DUX4-48.12 and DUX4-52.2), the band derived from DUX4-fl showed a marked decreases in its intensity.

### [Reference Example 1]

2-Cyanoethyl (6-stearamidohexyl)diisopropylphosphoramidite

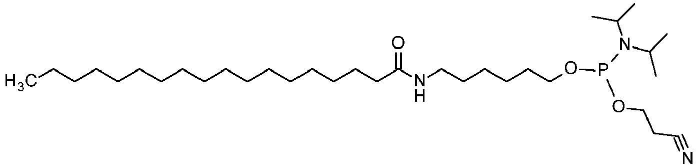

Stearic acid (5.00 g) suspension in dichloromethane (100 mL) was cooled to about 10°C. To this suspension, 6-amino-1-hexanol (3.09 g), 1-ethyl-3-(3-dimethylaminopropyl)carbodiimide hydrochloride (3.37 g) and diisopropylethylamine (3.06 mL) were added, and stirred for 17 hrs at room temperature. MeOH was added thereto. To the resultant residue upon concentration, acetone-MeOH-hexane-toluene and ISOLUTE HM-N (bulk, Biotage) were added for dispersion. The solvent was distilled off under vacuum. The residue obtained was subjected to silica gel column chromatography (hexane / ethyl acetate / methanol) to thereby obtain a crude product (3.98 g) of the intermediate. A suspension of this crude product (3.86 g) in dichloromethane (100 mL) was cooled to 0°C. To this suspension, diisopropylamine (7.01 mL) and 2-Cyanoethyl diisopropylchlorophosphoramidite (3.37 mL) were added and stirred for 3 hrs at room temperature. The solvent was distilled off under vacuum, and the residue was subjected to NH-silica gel column chromatography (DCM / ethyl acetate) and further to NH-silica gel chromatography (hexane / ethyl acetate) to thereby obtain the subject compound (785 mg) as a colorless solid.
¹H-NMR (CDCl₃) δ: 0.88 (3H, t, J = 6.3 Hz), 1.15-1.20 (12H, m), 1.22-1.66 (38H, m), 2.14 (2H, t, J = 7.6 Hz), 2.65 (2H, t, J = 6.3 Hz), 3.24 (2H, dt, J = 6.7, 6.7 Hz), 3.53-3.92 (6H, m), 5.44 (1H, br s). MS (m/z): 423.6 (M-N(iPr)₂+OH+Na)⁺.

### [Examples 131-134]

Compounds as shown in Table 4 below were synthesized under the same conditions as used in Example 1. It should be noted here that, after completion of the chain elongation of an oligonucleotide having the sequence of interest, 2-Cyanoethyl (6-palmitamidohexyl)diisopropylphosphoramidite (Nucleic Acids Res. (2020) 47, 6029-6044) was coupled to the oligonucleotide using a solution of this compound dissolved in acetonitrile : dichloromethane (1:2 v/v) to give a concentration of 0.1 M. The oligonucleotide after the coupling reaction was oxidized with an oxidizing solution [iodine solution (approx. 0.05 mol/L)] [pyridine : water (9:1)] (Sigma Aldrich).

The protected oligonucleotide analog with the sequence of interest was treated with 600 µl of concentrated aqueous ammonia to thereby cut out from the support and, at the same time, remove the protective group cyanoethyl on phosphorus atoms and the protective group on nucleobases. The resultant oligomer mixture in solution was mixed with 300 µl of Clarity QSP DNA Loading Buffer (Phenomenex) and charged on Clarity QSP Cartridge 60 mg/3 mL 30 µm (Phenomenex). One milliliter of Clarity QSP DNA Loading Buffer : water = 1:1 solution, 2 mL of 0.1 M tetrabutylammonium bromide aqueous solution : acetonitrile = 8:2 (v/v) solution, 4 mL of water, and 4 mL of 20 mM Tris aqueous solution were added in this order. Subsequently, components extracted with 800 µl of 8:2 solution of 20 mM Tris aqueous solution and acetonitrile were collected. After distilling off the solvent, the compound of interest was obtained. When analyzed by reversed-phase HPLC [column (Phenomenex, Clarity 2.6 µm Oligo-MS 100A (2.1x50 mm)), Solution A: an aqueous solution of 100 mM hexafluoroisopropanol (HFIP) and 8 mM trimethylamine, Solution B: methanol, B%: from 10% to 25% to 40% to 65% (4 min →76 min→8 min, linear gradient); 60°C; 0.5 mL/min; 260 nm)], the subject compound was eluted at 5.7 min. The compound was identified by negative-ion ESI mass spectrometry.

**[Table 4]**

| Example | Sequence Designation | Sequence (5'-3') | Target Region of DUX4-fl | | Molecular Weight (found) | SEQ ID NO |
|---|---|---|---|---|---|---|
| | | | 3' end position | 5' end position | | |
| 131 | DUX4-48.7 pal | CgaAggCgacccAcgAgg | 524 | 541 | 6858.10 | 23 |
| 132 | DUX4-48.10 pal | cgaAggcgaccCaCgAgg | 524 | 541 | 6846.10 | 23 |
| 133 | DUX4-48.11 pal | cgAaggcgAcCcacgAgg | 524 | 541 | 6832.07 | 23 |
| 134 | DUX4-52.1 pal | TgTgggCgaaggCgaCcC | 530 | 547 | 6920.08 | 100 |

In the sequences shown in the Table, small letters indicate 2'-OMe-RNA, and capital letters indicate ENA. It should be noted here that the nucleobase of C in ENA is 5-methylcytosine. All the bonds between nucleosides are phosphorothioate bonds. The target region is defined with nucleotide numbers of Homo sapiens clone 60-1 double homeodomain protein DUX4-fl (DUX4) mRNA (NCBI-GenBank accession No.HQ266761). The molecular weight indicates values actually found by negative-ion ESI mass spectrometry. The compounds of Examples 131-134 have a group represented by the following formula at the 5' end instead of a hydroxyl group.

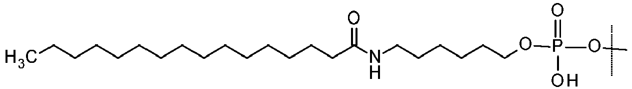

### [Examples 135-138]

Compounds as shown in Table 5 below were synthesized under the same conditions as used in Example 1. It should be noted here that, after completion of the chain elongation of an oligonucleotide having the sequence of interest, the compound of Reference Example 1 was coupled to the oligonucleotide using a solution of this compound dissolved in acetonitrile : dichloromethane (1:2 v/v) to give a concentration of 0.1 M. The oligonucleotide after the coupling reaction was oxidized with an oxidizing solution [iodine solution (approx. 0.05 mol/L)] [pyridine : water (9:1)] (Sigma Aldrich).

The protected oligonucleotide analog with the sequence of interest was treated with 600 µl of concentrated aqueous ammonia to thereby cut out from the support and, at the same time, remove the protective group cyanoethyl on phosphorus atoms and the protective group on nucleobases. The resultant oligomer mixture in solution was mixed with 300 µl of Clarity QSP DNA Loading Buffer (Phenomenex) and charged on Clarity QSP Cartridge 60 mg/3 mL 30 µL (Phenomenex). One milliliter of Clarity QSP DNA Loading Buffer : water = 1:1 solution, 2 mL of 0.1 M tetrabutylammonium bromide aqueous solution : acetonitrile = 8:2 (v/v) solution, 4 mL of water, and 4 mL of 20 mM Tris aqueous solution were added in this order. Subsequently, components extracted with 800 µl of 8:2 solution of 20 mM Tris aqueous solution and acetonitrile were collected. After distilling off the solvent, the compound of interest was obtained. When analyzed by reversed-phase HPLC [column (Phenomenex, Clarity 2.6 µm Oligo-MS 100A (2.1x50 mm)), Solution A: an aqueous solution of 100 mM hexafluoroisopropanol (HFIP) and 8 mM trimethylamine, Solution B: methanol, B%: from 10% to 25% to 40% to 65% (4 min →76 min→8 min, linear gradient); 60°C; 0.5 mL/min; 260 nm)], the subject compound was eluted at 6.2 min. The compound was identified by negative-ion ESI mass spectrometry.

**[Table 5]**

| Example | Sequence Designation | Sequence (5'-3') | Target Region of DUX4-fl | | Molecular Weight (found) | SEQ ID NO |
|---|---|---|---|---|---|---|
| | | | 3' end position | 5' end position | | |
| 135 | DUX4-48.7 ste | CgaAggCgacccAcgAgg | 524 | 541 | 6886.12 | 23 |
| 136 | DUX4-48.10 ste | cgaAggcgaccCaCgAgg | 524 | 541 | 6874.12 | 23 |
| 137 | DUX4-48.11 ste | cgAaggcgAcCcacgAgg | 524 | 541 | 6860.08 | 23 |
| 138 | DUX4-52.1 ste | TgTgggCgaaggCgaCcC | 530 | 547 | 6948.15 | 100 |

In the sequences shown in the Table, small letters indicate 2'-OMe-RNA, and capital letters indicate ENA. It should be noted here that the nucleobase of C in ENA is 5-methylcytosine. All the bonds between nucleosides are a phosphorothioate bond. The target region is defined with nucleotide numbers of Homo sapiens clone 60-1 double homeodomain protein DUX4-fl (DUX4) mRNA (NCBI-GenBank accession No.HQ266761). The molecular weight indicates values actually found by negative-ion ESI mass spectrometry. The compounds of Examples 135-138 have a group represented by the following formula at the 5' end instead of a hydroxyl group.

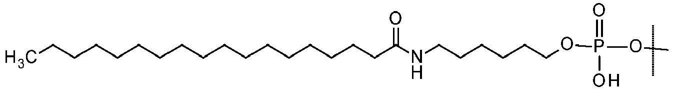

All publications, patents and patent applications cited herein are incorporated herein by reference in their entirety.

### INDUSTRIAL APPLICABILITY

The present invention is applicable to treatment of facioscapulohumeral muscular dystrophy.

### SEQUENCE LISTING FREE TEXT

### <SEQ ID NO: 1>

This sequence shows the nucleotide sequence of DUX4-fl accession number: HQ266761.

atggccctcccgacaccctcggacagcaccctccccgcggaagcccggggacgaggacggcgacggagactcgtttggaccccg
agccaaagcgaggccctgcgagcctgctttgagcggaacccgtacccgggcatcgccaccagagaacggctggcccaggccatcg
gcattccggagcccagggtccagatttggtttcagaatgagaggtcacgccagctgaggcagcaccggcgggaatctcggccctgg
cccgggagacgcggcccgccagaaggccggcgaaagcggaccgccgtcaccggatcccagaccgccctgctcctccgagccttt
gagaaggatcgctttccaggcatcgccgcccgggaggagctggccagagagacgggcctcccggagtccaggattcagatctggtt
tcagaatcgaagggccaggcacccgggacagggtggcagggcgcccgcgcaggcaggcggcctgtgcagcgcggcccccggc
gggggtcaccctgctccctcgtgggtcgccttcgcccacaccggcgcgtggggaacggggcttcccgcaccccacgtgccctgcgc
gcctggggctctcccacagggggctttcgtgagccaggcagcgagggccgcccccgcgctgcagcccagccaggccgcgccggc
agaggggatctcccaacctgccccggcgcgcggggatttcgcctacgccgccccggctcctccggacggggcgctctcccaccctc
aggctcctcgctggcctccgcacccgggcaaaagccgggaggaccgggacccgcagcgcgacggcctgccgggccccctgcgcg
gtggcacagcctgggcccgctcaagcggggccgcagggccaaggggtgcttgcgccacccacgtcccaggggagtccgtggtgg
ggctggggccggggtccccaggtcgccggggcggcgtgggaaccccaagccggggcagctccacctccccagcccgcgccccc
ggacgcctccgcctccgcgcggcaggggcagatgcaaggcatcccggcgccctcccaggcgctccaggagccggcgccctggtc
tgcactcccctgcggcctgctgctggatgagctcctggcgagcccggagtttctgcagcaggcgcaacctctcctagaaacggaggc
cccgggggagctggaggcctcggaagaggccgcctcgctggaagcacccctcagcgaggaagaataccgggctctgctggagga
gctttaggacgcggggttgggacggggtcgggtggttcggggcagggcggtggcctctctttcgcggggaacacctggctggctac
ggaggggcgtgtctccgccccgccccctccaccgggctgaccggcctgggattcctgccttctaggtctaggcccggtgagagactc
cacaccgcggagaactgccattctttcctgggcatcccggggatcccagagccggcccaggtaccagcagacctgcgcgcagtgcg
caccccggctgacgtgcaagggagctcgctggcctctctgtgcccttgttcttccgtgaaattctggctgaatgtctcccccaccttccg
acgctgtctaggcaaacctggattagagttacatctc

### <SEQ ID NOS: 2 to 85>

These show the sequences of the antisense oligonucleotides synthesized in Examples 1-118, 131-133 and 135-137. The antisense oligonucleotide may be either natural DNA, natural RNA, chimera DNA/RNA, or modified DNA, RNA or DNA/RNA, and at least one nucleotide constituting the antisense oligonucleotide may be a modified nucleotide.

### <SEQ ID NOS: 86 to 99>

These sequences show primer sequences.

### <SEQ ID NOS: 100 to 102>

These show the sequences of the antisense oligonucleotides synthesized in Examples 119-130 and 134-138. The antisense oligonucleotide may be either natural DNA, natural RNA, chimera DNA/RNA, or modified DNA, RNA or DNA/RNA, and at least one nucleotide constituting the antisense oligonucleotide may be a modified nucleotide.

## Claims

1. An oligonucleotide or a pharmaceutically acceptable salt thereof, wherein the oligonucleotide comprises an oligonucleotide of 15-30 bases consisting of a nucleotide sequence complementary to the region of nucleotide Nos. 502-556 or 578-612 of DUX4-fl mRNA consisting of the nucleotide sequence as shown in SEQ ID NO: 1; the 5' and/or 3' end of the oligonucleotide may be chemically modified; and the oligonucleotide is capable of switching the splice form of the DUX4 gene from DUX4-fl to DUX4-s.

2. The oligonucleotide or a pharmaceutically acceptable salt thereof of claim 1, wherein the oligonucleotide comprises a sequence of at least 15 consecutive nucleotides in any one of the sequences as shown in SEQ ID NOS: 2-85 (wherein "t" may be "u", and "u" may be "t").

3. The oligonucleotide or a pharmaceutically acceptable salt thereof of claim 1 or 2, wherein the oligonucleotide has 16-18 bases.

4. The oligonucleotide or a pharmaceutically acceptable salt thereof of claim 3, wherein the oligonucleotide has 18 bases.

5. The oligonucleotide or a pharmaceutically acceptable salt thereof of any one of claims 1 to 4, wherein at least one of the sugar and/or the phosphodiester bond constituting the oligonucleotide is modified.

6. The oligonucleotide or a pharmaceutically acceptable salt thereof of claim 5, wherein the sugar constituting the oligonucleotide is D-ribofuranose and modification of the sugar is modification of the hydroxy group at 2'-position of D-ribofuranose.

7. The oligonucleotide or a pharmaceutically acceptable salt thereof of claim 6, wherein modification of the sugar is 2'-O-alkylation and/or 2'-O,4'-C-alkylenation of D-ribofuranose.

8. The oligonucleotide or a pharmaceutically acceptable salt thereof of claim 6, wherein modification of the sugar is 2'-O-methylation and/or 2'-O,4'-C-ethylenation of D-ribofuranose.

9. The oligonucleotide or a pharmaceutically acceptable salt thereof of any one of claims 5 to 8, wherein modification of the phosphodiester bond is a phosphorothioate bond.

10. The oligonucleotide or a pharmaceutically acceptable salt thereof of any one of claims 1 to 9, wherein the oligonucleotide is one having 15-30 bases consisting of a nucleotide sequence complementary to the region of nucleotide Nos. 506-549 of the nucleotide sequence as shown in SEQ ID NO: 1.

11. The oligonucleotide or a pharmaceutically acceptable salt thereof of claim 10, wherein the oligonucleotide comprises a sequence of at least 15 consecutive nucleotides in any one of the sequences as shown in SEQ ID NOS: 5-31 (wherein "t" may be "u", and "u" may be "t").

12. An oligonucleotide consisting of any one of the following sequences or a pharmaceutically acceptable salt thereof:
HO-G^{mls}-G^{e2s}-G^{mls}-A^{mls}-G^{e2s}-C^{mls}-A^{mls}-G^{e2s}-G^{mls}-G^{mls}-T^{e2s}-G^{mls}-A^{mls}-C^{e2s}-C^{mls}-C^{mls}-C^{e2s}-C^{mlt}-H (DUX4-006);
HO-G^{mls}-A^{e2s}-C^{mls}-C^{mls}-C^{e2s}-A^{mls}-C^{mls}-G^{e2s}-A^{mls}-G^{mls}-G^{e2s}-G^{mls}-A^{mls}-G^{e2s}-C^{mls}-A^{mls}-G^{e2s}-G^{mlt}-H (DUX4-009);
HO-G^{mls}-A^{e2s}-A^{mls}-G^{mls}-G^{e2s}-C^{mls}-G^{mls}-A^{e2s}-C^{mls}-C^{mls}-C^{e2s}-A^{mls}-C^{mls}-G^{e2s}-A^{mls}-G^{mls}-G^{e2s}-G^{mlt}-H (DUX4-011);
HO-G^{mls}-G^{e2s}-U^{mls}-G^{mls}-T^{e2s}-G^{mls}-G^{mls}-G^{e2s}-C^{mls}-G^{mls}-A^{e2s}-A^{mls}-G^{mls}-G^{e2s}-C^{mls}-G^{mls}-A^{e2s}-C^{mlt}-H (DUX4-014);
HO-G^{mls}-A^{e2s}-G^{mls}-C^{mls}-A^{e2s}-G^{mls}-G^{mls}-G^{e2s}-U^{mls}-G^{mls}-A^{e2s}-C^{mls}-C^{mls}-C^{e2s}-C^{mls}-C^{mls}-G^{e2s}-C^{mlt}-H (DUX4-036);
HO-G^{mls}-G^{e2s}-A^{mls}-G^{mls}-C^{e2s}-A^{mls}-G^{mls}-G^{e2s}-G^{mls}-U^{mls}G^{e2s}-A^{mls}-C^{mls}-C^{e2s}-C^{mls}-C^{mls}-C^{e2s}-G^{mlt}-H (DUX4-037);
HO-A^{mls}-C^{e2s}-G^{mls}-A^{mls}-G^{e2s}-G^{mls}-G^{mls}-A^{e2s}-G^{mls}-C^{mls}-A^{e2s}-G^{mls}-G^{mls}-G^{e2s}-U^{mls}-G^{mls}-A^{e2s}-C^{mlt}-H (DUX4-040);
HO-C^{mls}-G^{e2s}-A^{mls}-C^{mls}-C^{e2s}-C^{mls}-A^{mls}-C^{e2s}-G^{mls}-A^{mls}-G^{e2s}-G^{mls}-G^{mls}-A^{e2s}-G^{mls}-C^{mls}-A^{e2s}-G^{mlt}-H (DUX4-044);
HO-A^{mls}-A^{e2s}-G^{mls}-G^{mls}-C^{e2s}-G^{mls}-A^{mls}-C^{e2s}-C^{mls}-C^{mls}-A^{e2s}-C^{mls}-G^{mls}-A^{e2s}-G^{mls}-G^{mls}-G^{e2s}-A^{mlt}-H (DUX4-047);
HO-C^{mls}-G^{e2s}-A^{mls}-A^{mls}-G^{e2s}-G^{mls}-C^{mls}-G^{e2s}-A^{mls}-C^{mls}-C^{e2s}-C^{mls}-A^{mls}-C^{e2s}-A^{mls}-A^{mls-}G^{e2s}-G^{mlt}-H (DUX4-048);
HO-G^{mls}-C^{e2s}-G^{mls}-A^{mls}-A^{e2s}-G^{mls}-G^{mls}-C^{e2s}-G^{mls}-A^{mls}-C^{e2s}-C^{mls}-C^{mls}-A^{e2s}-C^{mls}-G^{mls}-A^{e2s}-G^{mlt}-H (DUX4-049);
HO-U^{mls}-G^{e2s}-U^{mls}-G^{mls}-G^{e2s}-G^{mls}-C^{mls}-G^{e2s}-A^{mls}-A^{mls}-G^{e2s}-G^{mls}-C^{mls}-G^{e2s}-A^{mls}-C^{mls}-C^{e2s}-C^{mlt}-H (DUX4-052);
HO-G^{mls}-T^{e2s}-G^{mls}-U^{mls}-G^{e2s}-G^{mls}-G^{mls}-C^{e2s}-G^{mls}-A^{mls}-A^{e2s}-G^{mls}-G^{mls}-C^{e2s}-G^{mls}-A^{mls}-C^{e2s}-C^{mlt}-H (DUX4-053);
HO-C^{e2s}-G^{mls}-A^{mls}-A^{e2s}-G^{mls}-G^{mls}-C^{e2s}-G^{mls}-A^{mls}-C^{mls}-C^{mls}-C^{mls}-A^{e2s}-C^{mls}-G^{mls}-A^{e2s}-G^{mls}-G^{mlt}-H (DUX4-48.7);
HO-C^{mls}-G^{mls}-A^{mls}-A^{e2s}-G^{mls}-G^{mls}-C^{mls}-G^{mls}-A^{mls}-C^{mls}-C^{mls}-C^{e2s}-A^{mls}-C^{e2s}-G^{mls}-A^{e2s}-G^{mls}-G^{mlt}-H (DUX4-48.10);
HO-C^{mls}-G^{mls}-A^{e2s}-A^{mls}-G^{mls}-G^{mls}-C^{mls}-G^{mls}-A^{e2s}-C^{mls}-C^{e2s}-C^{mls}-A^{mls}-C^{mls}-G^{mls}-A^{e2s}-G^{mls}-G^{mlt}-H (DUX4-48.11)
HO-C^{e2s}-G^{mls}-A^{e2s}-A^{mls}-G^{mls}-G^{mls}-C^{mls}-G^{mls}-A^{mls}-C^{mls}-C^{mls}-C^{mls}-A^{mls}-C^{e2s}-G^{mls}-A^{e2s}-G^{mls}-G^{mlt}-H (DUX4-48.12);
HO-C^{mls}-G^{mls}-A^{mls}-A^{e2s}-G^{mls}-G^{mls}-C^{mls}-G^{mls}-A^{mls}-C^{e2s}-C^{mls}-C^{mls}-A^{mls}-C^{e2s}-G^{mls}-A^{e2s}-G^{mls}-G^{mlt}-H (DUX4-48.14);
HO-C^{mls}-G^{mls}-A^{mls}-A^{e2s}-G^{mls}-G^{mls}-C^{mls}-G^{mls}-A^{e2s}-C^{mls}-C^{mls}-C^{mls}-A^{mls}-C^{e2s}-G^{mls}-A^{e2s}-G^{mls}-G^{mlt}-H (DUX4-48.15);
HO-C^{mls}-G^{mls}-A^{e2s}-A^{mls}-G^{mls-}G^{mls}-C^{e2s}-G^{mls}-A^{mls}-C^{e2s}-C^{mls}-C^{mls}-A^{mls}-C^{mls}-C^{mls}-A^{e2s}-G^{mls}-G^{mlt}-H (DUX4-48.19);
HO-C^{mls}-G^{mls}-A^{mls}-A^{e2s}-G^{mls}-G^{mls}-C^{mls}-G^{mls}-A^{mls}-C^{mls}-C^{mls}-C^{mls}-A^{mls}-C^{e2s}-G^{mls}-A^{e2s}-G^{mls}-G^{mlt}-H (DUX4-48.20);
HO-T^{e2s}-G^{mls}-T^{e2s}-G^{mls}-G^{mls}-G^{mls}-C^{e2s}-G^{mls}-A^{mls}-A^{mls}-G^{mls}-G^{mls}-C^{e2s}-G^{mls}-A^{mls}-C^{e2s}-C^{mls}-C^{e2t}-H (DUX4-52.1);
HO-T^{e2s}-G^{mls}-T^{e2s}-G^{mls}-G^{mls}-G^{mls}-C^{e2s}-G^{mls}-A^{mls}-A^{e2s}-G^{mls}-G^{mls-}C^{e2s}-G^{mls}-A^{mls}-C^{e2s}-C^{mls}-C^{mlt}-H (DUX4-52.2);
HO-T^{e2s}-G^{mls}-U^{mls}-G^{mls}-G^{mls}-G^{mls}-C^{mls}-G^{mls}-A^{mls}-A^{mls}-G^{mls}-G^{mls}-^{Ce2s}-G^{mls}-A^{mls}-C^{e2s}-C^{mls}-C^{e2t}-H (DUX4-52.7);
HO-U^{mls}-Gml^{s}-T^{e2s}-G^{mls}-G^{mls}-G^{mls}-C^{mls}-G^{mls}-A^{mls}-A^{mls}-G^{mls}-G^{mls}-C^{e2s}-G^{mls}-A^{e2s}-C^{mls}-C^{e2s}-C^{mlt}-H (DUX4-52.9)
[wherein A^{e2s}, G^{e2s}, C^{e2s} and T^{e2s} represent corresponding ENAs (where the nucleobase of C is 5-methylcytosine) binding to a structure adjacent to the 3' side by a phosphorothioate bond; A^{mls}, G^{mls}, C^{mls} and U^{mls} represent corresponding 2'-OMe-RNAs binding to a structure adjacent to the 3' side by a phosphorothioate bond; C^{e2t} represents corresponding ENA (where the nucleobase of C is 5-methylcytosine) binding to a structure adjacent to the 3' side by a phosphodiester bond; and A^{mlt}, G^{mlt} and C^{mlt} represent corresponding 2-OMe-RNAs binding to a structure adjacent to the 3' side by a phosphodiester bond].

13. The oligonucleotide or a pharmaceutically acceptable salt thereof of any one of claims 1 to 12 above, wherein an aminoalkylphosphate group containing a fatty acid(s) is further bound at the 5' or 3' end of the oligonucleotide.

14. The oligonucleotide or a pharmaceutically acceptable salt thereof of claim 13, wherein the fatty acid is at least one selected from the group consisting of myristic acid, palmitic acid, stearic acid, arachidic acid and behenic acid.

15. The oligonucleotide or a pharmaceutically acceptable salt thereof of any one of claims 1 to 14, for use in treatment of a disease or a symptom attributable to DUX4-fl expression.

16. The oligonucleotide or a pharmaceutically acceptable salt thereof of claim 15, wherein the disease or symptom attributable to DUX4-fl expression is facioscapulohumeral muscular dystrophy.

17. A pharmaceutical drug comprising the oligonucleotide or a pharmaceutically acceptable salt thereof of any one of claims 1 to 16.

18. A therapeutic for a disease or a symptom attributable to DUX4-fl expression, which comprises the oligonucleotide or a pharmaceutically acceptable salt thereof of any one of claims 1 to 16.

19. The therapeutic of claim 18, wherein the disease or symptom attributable to DUX4-fl expression is facioscapulohumeral muscular dystrophy.

20. A agent capable of switching the splice form of the DUX4 gene from DUX4-fl to DUX4-s, which comprises the oligonucleotide or a pharmaceutically acceptable salt thereof of any one of claims 1 to 16.

21. A method of treating a disease or a symptom attributable to DUX4-fl expression in a subject, comprising administering to the subject the oligonucleotide or a pharmaceutically acceptable salt thereof of any one of claims 1 to 16.

22. The method of claim 21, wherein the disease or symptom attributable to DUX4-fl expression is facioscapulohumeral muscular dystrophy.

23. Use of the oligonucleotide or a pharmaceutically acceptable salt thereof of any one of claims 1 to 16, for manufacturing a therapeutic for a disease or a symptom attributable to DUX4-fl expression.

24. The use of claim 23 above, wherein the disease or symptom attributable to DUX4-fl expression is facioscapulohumeral muscular dystrophy.
